(19) Europäisches Patentamt
European Patent Office
Office européen des brevets

(11) EP 4 148 033 B1

(12) EUROPEAN PATENT SPECIFICATION

(45) Date of publication and mention of the grant of the patent:
**31.12.2025 Bulletin 2026/01**

(21) Application number: **21825888.7**

(22) Date of filing: **26.01.2021**

(51) International Patent Classification (IPC):
***C07C 9/00*** *(2006.01)* ***C09K 3/00*** *(2006.01)*
***B01D 53/79*** *(2006.01)* ***C10L 3/10*** *(2006.01)*
***B01D 53/62*** *(2006.01)*

(52) Cooperative Patent Classification (CPC):
**C10L 3/108**

(86) International application number:
**PCT/CN2021/073733**

(87) International publication number:
**WO 2021/253833 (23.12.2021 Gazette 2021/51)**

# (54) HYDRATE ACCELERATOR, HYDRATE AND PREPARATION METHOD THEREFOR

HYDRATBESCHLEUNIGER, HYDRAT UND HERSTELLUNGSVERFAHREN DAFÜR

ACCÉLÉRATEUR D’HYDRATE, HYDRATE ET PROCÉDÉ DE PRÉPARATION ASSOCIÉ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **18.06.2020 CN 202010564049**
**18.06.2020 CN 202010562124**
**18.06.2020 CN 202010562090**
**18.06.2020 CN 202010561406**
**18.06.2020 CN 202010561395**

(43) Date of publication of application:
**15.03.2023 Bulletin 2023/11**

(73) Proprietors:
- **China Petroleum & Chemical Corporation**
**Beijing 100728 (CN)**
- **Sinopec Research Institute of Safety Engineering Co., Ltd.**
**Qingdao, Shandong 266104 (CN)**

(72) Inventors:
- **YAN, Kele**
**Qingdao, Shandong 266104 (CN)**
- **XIAO, Anshan**
**Qingdao, Shandong 266104 (CN)**
- **MU, Shanjun**
**Qingdao, Shandong 266104 (CN)**
- **HU, Xuyao**
**Qingdao, Shandong 266104 (CN)**
- **REN, Yuemeng**
**Qingdao, Shandong 266104 (CN)**
- **ZHANG, Hongxing**
**Qingdao, Shandong 266104 (CN)**
- **LIN, Yu**
**Qingdao, Shandong 266104 (CN)**

(74) Representative: **Hoffmann Eitle**
**Patent- und Rechtsanwälte PartmbB**
**Arabellastraße 30**
**81925 München (DE)**

(56) References cited:
**CN-A- 1 428 082** **CN-A- 101 721 366**
**CN-A- 106 914 121** **CN-A- 108 770 836**
**CN-A- 109 266 312** **CN-A- 109 908 079**
**CN-B- 106 634 827** **RU-C1- 2 609 824**
**US-A1- 2005 261 529** **US-A1- 2007 248 527**
**US-A1- 2015 147 279**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 148 033 B1

## Description

## FIELD

The present disclosure pertains to the technical field of safe storage and transportation of natural gas, and particularly relates to a hydrate promoter, a hydrate and a preparation method thereof.

## BACKGROUND

The technology of hydrate method storage and transportation of natural gas (SNG) serves to perform safe storage and transportation of natural gas by forming solid crystal compounds with water under mild conditions. Theoretically, 1 $m^3$ of hydrate can store 150-180 $m^3$ of methane. It is characterized in that: (1) the preparation and storage conditions are mild; for example, the formation pressure of hydrate at 8°C is less than 3.0 MPa, it is reported in the literatures that the atmospheric pressure storage and transportation can be performed under the temperature of -5°C to -10°C; (2) the storage and transportation process is safe and reliable; the decomposition rate of a simple gas hydrate is extremely slow due to the self-protective effect of hydrate, and water phase generated during the decomposition process causes that the risk of explosion in the storage and transportation process is greatly reduced; (3) the gas quality requirement is low; the hydrate is insensitive to impurities such as water, heavy hydrocarbons, $CO_2$ and $H_2S$, the impurities do not affect formation of the hydrate; (4) the application fields of hydrate storage and transportation technology are widespread.

CN101672425A with an application number 200810156914.1 discloses a composite hydrate promoter, the composite hydrate promoter is prepared by mixing cyclopentane or methyl cyclohexane serving as a kinetic promoter and 2-butyl octyl sodium sulfate serving as a surfactant. However, this type of promoter has a long induction time and a limited gas storage capacity.

CN103028353A with an application number 201210544097.3 discloses a preparation method of a degradable gas hydrate efficient promoter, the promoter is prepared by three substances including rhamnolipid, sodium carbonate ($Na_2CO_3$) and sodium chloride (NaCl) according to a certain ratio. However, this type of promoter has a limited ability to enhance the generation rate of hydrate, and the patent literature merely formulates the promotion effect of the $CO_2$ hydrate. CN106634827A with an application number 201610813750.X discloses a composite gas hydrate promoter, the promoter is composed of a twelve-alkyl hydroxypropyl phosphate betaine as an additive and alkyl phosphate salts and octadecylamine propyl oxamide as surface active agents, based on the total volume of the mixed aqueous solution, the mass percentage content of the twelve-alkyl hydroxypropyl phosphate betaine is 1 to 2.5%; the mass percentage content of the surface active agent alkyl phosphate salt is 1 to 3.5%; the mass percentage content of the surface active agent octadecylamine propyl oxamide is 2.5 to 4%; and the remainder is deionized water.

As can be seen, the currently available hydrate promoter still suffers from the disadvantages such as low generation rate of hydrate. Therefore, it is the problem shall be urgently solved in the art to provide a hydrate promoter that increases generation rate and gas storage capacity of the hydrate.

## SUMMARY

The present disclosure aims to solve the problems in the prior art of hydrate promoter concerning the low generation rate and small gas storage capacity of hydrate, and provides a hydrate promoter, a hydrate and a method for preparing the hydrate, wherein the hydrate promoter is capable of increasing the generation rate and the gas storage capacity of the hydrate.

In order to fulfill the above purpose, a first aspect of the present disclosure provides a hydrate promoter, comprising a component A which is a substance having a group represented by the Formula I below and a surfactant, wherein a molar ratio of the component A to the surfactant is 1: (0.03-30);

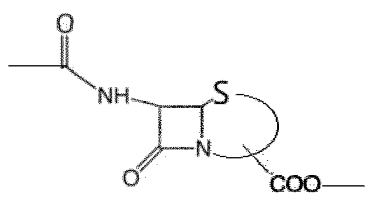

I ,

wherein the reference signs "-" marked by 1, 2 in Formula I are chemical bonds; the cyclic structure having N and S in

Formula I is a 4-8 member ring, the group -COO-is located at the ortho-position or meta-position relative to the N atom of the ring in the cyclic structure having N and S; the remaining backbone atoms of the cyclic structure having N and S in Formula I are C atoms; wherein, the component A is selected from the penicillin compounds, and the surfactant is consisting of a betaine type zwitterionic surfactant and a fatty alcohol polyoxyethylene ether non-ionic surfactant; wherein a mass ratio of the betaine type zwitterionic surfactant to the fatty alcohol polyoxyethylene ether non-ionic surfactant is (0.1-10):1.

In a second aspect, the present disclosure provides a method for preparing a hydrate, the method comprises contacting a gas in an aqueous system with the hydrate promoter of the first aspect under hydrate formation conditions.

In a third aspect, the present disclosure provides a hydrate comprising the hydrate promoter of the first aspect.

In the above-mentioned technical schemes, the hydrate promoter comprising a component A of a substance having the group represented by Formula I and a surfactant exhibits significant promotion effect, the hydrate promoter can effectively increase generation rate of hydrate and improve the gas storage capacity. In addition, the inventors of the present disclosure have discovered in research that hydrate promoter in the prior art not only has a problem of small gas storage capacity of hydrate, but also generates a large amount of air bubbles during the decomposition process of hydrate, such that gas recovery is hindered and gas recovery rate is low; on the contrary, the hydrate promoter of the present disclosure not only has high gas storage capacity, but also can inhibit the generation of air bubbles during the decomposition process of hydrate, provide a high gas recovery rate. The reasons may be that the hydrate formed by the hydrate promoter of the present disclosure can promote nucleation and growth of hydrate along the wall of a reactor, and maintain the surface tension of water phase at a level close to that of pure water phase during the decomposition process of hydrate, thereby inhibiting the generation of air bubbles during the decomposition process of hydrate, increasing the decomposition efficiency of hydrate and the gas recovery rate. The present disclosure desirably overcomes the disadvantages of the conventional hydrate promoter such as small gas storage capacity and low generation rate of hydrate, the present disclosure is further capable of suppressing generation of air bubbles and improving gas recovery rate of the hydrate during decomposition process of the hydrate, as compared to the conventional hydrate promoter, thus has a favorable application prospect for natural gas storage and transportation with the hydrate.

It has been confirmed that by applying the hydrate promoter of the present disclosure, the induction time of hydrate can be merely 1.5min when the generation pressure of hydrate is 7.0MPa; moreover, no obvious air bubbles appear during the decomposition process of hydrate, and the gas recovery rate reaches 97% after completion of the decomposition process, and it is discovered by further detection that the surface tension (at a temperature of 298.15K) of the aqueous phase after the decomposition of hydrate is 68mN/m, which is proximate to the surface tension (71mN/m) of the pure water phase.

## BRIEF DESCRITION OF THE DRAWINGS

FIG. 1 illustrates a graph showing changes in the system pressure during the generation process of a hydrate in Comparative Examples 1-3 and Example 1.

FIG. 2 illustrates a graph showing changes in gas storage capacity during the generation process of a hydrate in in Comparative Examples 1-3 and Example 1.

FIG. 3 illustrates the conditions of generating air bubbles in the autoclave after the decomposition of hydrate in Comparative Examples 1-3 and Example 1.

## DETAILED DESCRPITION

The terminals and any value of the ranges disclosed herein are not limited to the precise ranges or values, such ranges or values shall be comprehended as comprising the values adjacent to the ranges or values. As for numerical ranges, the endpoint values of the various ranges, the endpoint values and the individual point value of the various ranges, and the individual point values may be combined with one another to produce one or more new numerical ranges, which should be deemed have been specifically disclosed herein.

In a first aspect, the present disclosure provides a hydrate promoter, comprising a component A which is a substance having a group represented by the Formula I below and a surfactant, wherein a molar ratio of the component A to the surfactant is 1: (0.03-30);

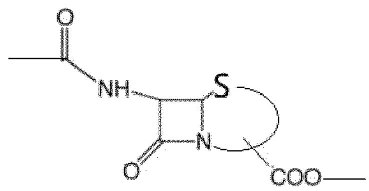

I ,

wherein the reference signs "-" marked by 1, 2 in Formula I are chemical bonds; the cyclic structure having N and S in Formula I is a 4-8 member ring, the group -COO- is located at the ortho-position or meta-position relative to the N atom of the ring in the cyclic structure having N and S; the remaining backbone atoms of the cyclic structure having N and S in Formula I are C atoms; wherein, the component A is selected from the penicillin compounds, and the surfactant is consisting of a betaine type zwitterionic surfactant and a fatty alcohol polyoxyethylene ether non-ionic surfactant; wherein a mass ratio of the betaine type zwitterionic surfactant to the fatty alcohol polyoxyethylene ether non-ionic surfactant is (0.1-10):1.

The inventors of the present disclosure have discovered in research that hydrate promoter in the prior art not only has a problem of low generation rate and small gas storage capacity of hydrate, but also frequently generates a large amount of air bubbles during the decomposition process of hydrate, such that the gas recovery is hindered and the gas recovery rate is low; on the contrary, the hydrate promoter components provided by the present disclosure comprise a penicillin compound and/or a cephalosporin compound, (the latter are not according to the invention) it can have a high generation rate of hydrate and a high gas recovery rate.

According to the present disclosure, the molar ratio of the component A and the surfactant is 1: (0.03-30), and the molar ratio may be a ratio of 1 relative to the following numerical values, for example, 0.03, 0.1, 0.2, 0.3, 0.4, 0.5, 0.6, 0.7, 0.8, 1.5, 2, 2.5, 3, 3.5, 4, 4.5, 5, 5.5, 6, 6.5, 7, 7.5, 8, 8.5, 9, 9.5, 10, 11, 12, 13, 14, 15, 16, 17, 18, 19, 20, 21, 22, 23, 24, 25, 26, 27, 28, 29, 30, and a ratio between 1 and a random value between any two numerical values of 0.03-30.

According to the present disclosure, with respect to the group represented by

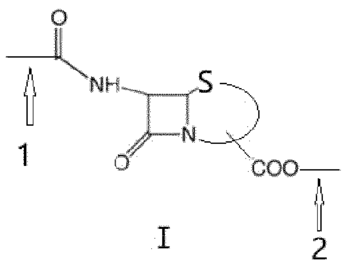

I

Formula I ,

the reference signs "-" marked by 1, 2 are chemical bonds, instead of methyl.

In order to further reduce the induction time of hydrate, further increase the nucleation and formation rate of hydrate, further suppress the generation of air bubbles during the decomposition process of hydrate, and further increase the gas recovery rate of hydrate, a mass ratio of the component A to the surfactant is preferably 1: (0.1-10), and the mass ratio may be, for example, a ratio of 1 relative to the following numerical values: 0.1, 0.5, 1, 2, 3, 4, 5, 6, 7, 8, 9, or 10, and a ratio between 1 and a random value between any two numerical values of 0.1-10. Preferably, the mass ratio of the component A to the surfactant is 1: (0.1-5); the mass ratio may be, for example, a ratio of 1 relative to the following numerical values: 0.1, 0.3, 0.9, 1.2, 1.5, 1.8, 2.1, 2.5, 3.0, 3.5, 4.0, 4.5, or 5, and a ratio between 1 and a random value between any two numerical values of 0.1-5.

In accordance with the present disclosure, besides the group -COO-, the cyclic structure having N and S in Formula I may have other substituted groups, and the positions of substitution may have many options, the desired inventive effect can be obtained as long as the substance having the group represented by Formula I and the surfactant are combined in a specific ratio according to the present disclosure. For the substance having the group represented by Formula I, the number of backbone atoms in the cyclic structure having N and S in Formula I may have many options, for example, 4-8 member ring, i.e., 4 member ring, 5 member ring, 6 member ring, 7 member ring, 8 member ring, The substitution position is an ortho-position or a meta-position relative to the N atom of the ring in the cyclic structure having N and S. In order to further shorten the induction time of hydrate, further improve the nucleation and formation rate of hydrate, further suppress the generation of air bubbles during the decomposition process of hydrate, and further improve the gas recovery rate of hydrate, the cyclic structure having N and S in Formula I is a 4-8 member ring, the group -COO- is located at

the ortho-position or meta-position relative to the N atom of the ring in the cyclic structure having N and S.

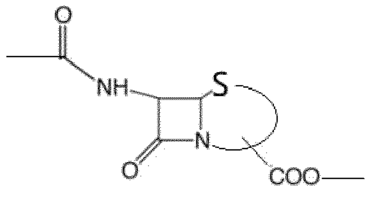

I

According to the present disclosure, the cyclic structure having N and S in Formula I comprises C, N and S shown in Formula I, the remaining backbone atoms are backbone atom C C-C in Formula I can be present either as a saturated bond or an unsaturated bond, and further preferably, the cyclic structure having N and S in Formula I is a saturated 4-8 member ring or a 4-8 member ring having 1-3 C-C unsaturated bonds.

According to the present disclosure, in order to further reduce the induction time of hydrate, further increase the nucleation and formation rate of hydrate, further suppress the generation of air bubbles during the decomposition process of hydrate, and further increase the gas recovery rate of hydrate, it is preferred that the cyclic structure having N and S in Formula I is a 5-6 member ring, and the remaining backbone atoms of the cyclic structure having N and S are C atom. The group -COO- is located on a carbon atom at the ortho-position or meta-position relative to the N atom of the 5-6 member ring, preferably, the group -COO- is located at an ortho-position relative to the N atom of the ring in the cyclic structure having N and S.

Further preferably, the component A is selected in reference embodiments from cephalosporins compound having a group represented by Formula II and in inventive embodiments from penicillins compound having a group represented by

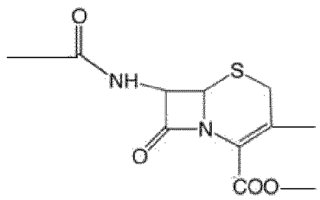

Formula III.

II

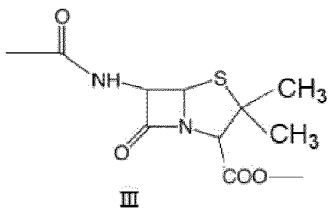

III

In the preferred embodiment, the hydrate promoter of the present disclosure can further reduce the induction time of hydrate, further increase the nucleation and formation rate of hydrate, further suppress the generation of air bubbles during the decomposition process of hydrate, and further increase the gas recovery rate of hydrate. The reasons may be that the hydrogenated thiazole ring in the molecular structure of penicillin or in reference embodiments the hydrogenated thiazine ring of cephalosporin compounds of the present disclosure can serve as hydrophobic group to increase the contact with gas and promote the dissolution of gas at the gas-liquid interface; in the other hand, the nitrogen and sulfur heteroatoms contained in the hydrogenated thiazole ring or the hydrogenated thiazine ring have rich electrons, which are apt to form hydrogen bonds with water, further increase the action strength of water and organic gas (methane) at the gas-liquid interface, thereby promoting the formation of hydrate; in combination with characteristic that the walls of reactor are conducive to heat transfer, such that the formed hydrate can promote the nucleation and growth of hydrate along the walls of the reactor, and maintain the surface tension of the water phase at a level close to that of the pure water phase during the decomposition process of hydrate, thereby inhibiting the generation of air bubbles during the decomposition process of hydrate, increasing the decomposition efficiency and gas recovery of hydrate. The present disclosure desirably overcomes the disadvantages of the conventional hydrate promoter such as small gas storage capacity and low generation

rate, the present disclosure can effectively reduce the time for hydrate induction, significantly improve the rate of hydrate nucleation and formation, and can suppress generation of air bubbles and improve gas recovery rate of the hydrate during decomposition process of the hydrate, as compared to the conventional hydrate promoter, thus has a favorable application prospect for natural gas storage and transportation with the hydrate.

According to the present disclosure, with respect to the groups represented by

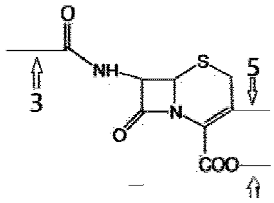

Formula II

(not part of the invention) 4 , the reference signs "-" marked by 3, 4, 5 are chemical bonds, instead of methyl; with respect to the groups represented by

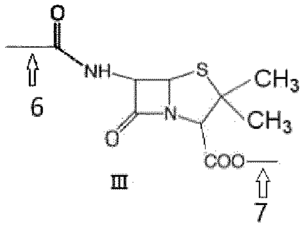

III

Formula III ,

the reference signs "-" marked by 6, 7 are chemical bonds, instead of methyl.

According to reference embodiments, it is still further preferred that the component A is a cephalosporin compound, (not part of the invention) a mass ratio of the cephalosporin compound to the surfactant is preferably 1: (0.1-5), the mass ratio may be, for example, a ratio of 1 relative to the following numerical values: 0.1, 0.3, 0.9, 1.2, 1.5, 1.8, 2.1, 2.5, 3.0, 3.5, 4.0, 4.5, or 5, and a ratio between 1 and a random value between any two numerical values of 0.1-5.

Cephalosporins and penicillins both pertain to the beta-lactam antibiotic compounds, except that parent nucleus of the cephalosporin antibiotic is 7-aminocephalosporanic acid (7-ACA), i.e., the group -COO-in

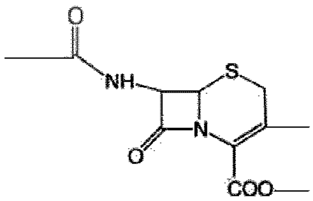

Formula II

II

is replaced by the group -COOH. The cephalosporin compound of reference embodiments mainly serve to promote nucleation and growth of hydrate. The cephalosporin compound is selected from the group consisting of a cephalosporin antibiotic and/or a salt corresponding to the cephalosporin antibiotic. There are various options for the salt corresponding to the cephalosporin antibiotic. Preferably, the salt corresponding to the cephalosporin antibiotic is at least one selected from the group consisting of sodium salt, potassium salt and ammonium salt.

The cephalosporin antibiotic of reference embodiments is the partially synthesized antibacterial drug by connecting the cephalosporin core 7-aminocephalosporanic acid (7-ACA) with different chains. The cephalosporin drugs are currently divided into five generations according to their antibacterial spectrum, antibacterial activity, stability against β-lactamase and nephrotoxicity. In accordance with the present disclosure, the desirable results can be produced as long as they belong to the cephalosporin antibiotic and/or the salt corresponding to the cephalosporin antibiotic. Preferably, the cephalosporin antibiotic is at least one selected from the group consisting of ceftiomib, cefamandole, cefadroxil, cefadroxime, hydroxamitocetin, cefchlorampin, cefalexin, cephalexin monohydrate and cefotaxime; still further preferably, the cephalosporin antibiotic is at least one selected from the group consisting of cefadroxil, cephalexin, cefchlorampin, cefalexin and cefotaxime.

The molecular structural formula of a portion of the cephalosporin antibiotics are shown in the attached Table 1.

Table 1

| Cephalosporin compounds of reference embodiments | Molecular structural formula |
| --- | --- |
| Mono-hydrate cefchlorampin | 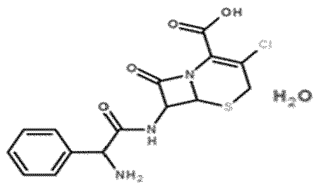 |
| Cefalexin | 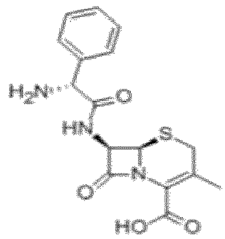 |
| Cefotaxime | 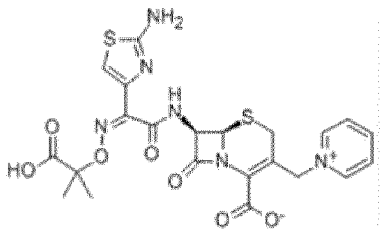 |
| Cefadroxil | 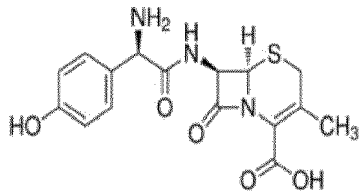 |
| Cephalexin monohydrate | 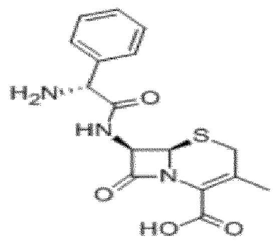 |

According to the present disclosure, the penicillin compound is selected from the group consisting of penicillin antibiotics and/or a salt corresponding to the penicillin antibiotic. There are various options for the salt corresponding to the penicillin antibiotic, preferably, the salt corresponding to the penicillin antibiotic is at least one selected from the group consisting of sodium salt, potassium salt and ammonium salt.

According to the present disclosure, it is further preferred that when the component A is a penicillin compound, a mass ratio of the penicillin compound to the surfactant is preferably 1: (0.1-5), further preferably 1: (0.1-3), the mass ratio may be, for example, a ratio of 1 relative to the following numerical values: 0.2, 0.4, 0.6, 0.8, 1.0, 1.2, 1.4, 1.6, 1.8, 2.0, 2.2, 2.4, 2.6, 2.8, 3, and a ratio between 1 and a random value between any two numerical values of 0.1-3. Still further preferably, the mass ratio of the penicillin compound to the surfactant is 1: (0.1-1).

According to the present disclosure, parent nucleus of the penicillin antibiotics is 6-aminopenicillanic acid, i.e. the group -COO- in

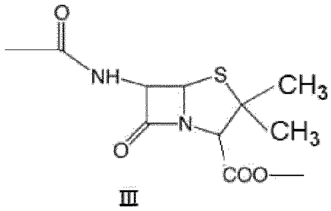

III

Formula III

is replaced by the group -COOH, and the penicillin antibiotics are phenylacetyl derivatives of 6-aminopenicillanic acid. According to the present disclosure, the penicillin antibiotic is at least one selected from the group consisting of penicillin G class, penicillin V class, enzyme resistant penicillin, ampicillin class and pseudomonas resistant penicillin; for example, the penicillin compound can be penicillin G, such as penicillin G potassium, penicillin G sodium, tardocillin-penicillin G, penicillin, penicillin sodium, benzyl penicillin sodium, penicillin potassium, benzyl penicillin potassium; or penicillin V, such as phenoxymethyl penicillin, 6-phenoxyacetamidolevulanic acid, penicillin V potassium; or enzyme resistant penicillin: such as benzylpenicillin (oxacillin), cloxacillin; or ampicillins, such as ampicillin, amoxicillin; or pseudomonas resistant penicillin, such as carbenicillin, piperacillin, ticarcillin.

In order to further promote the nucleation and growth of hydrate, thereby further reducing the induction time of hydrate and further increasing the growth rate of hydrate, and further improving the gas recovery rate during the decomposition process of hydrate, preferably, the penicillin compound is at least one selected from the group consisting of ampicillin sodium, ampicillin, penicillin potassium, carbenicillin sodium, penicillin sodium and oxacillin sodium.

Wherein the molecular structural formula of some penicillin compounds are shown in Table 2.

Table 2

| Penicillin compounds | Molecular structural formula |
|---|---|
| Penicillin sodium | 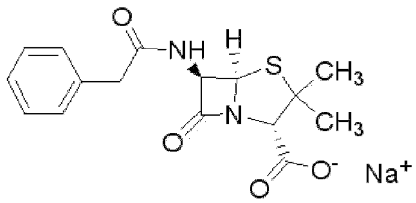 |
| Penicillin potassium | 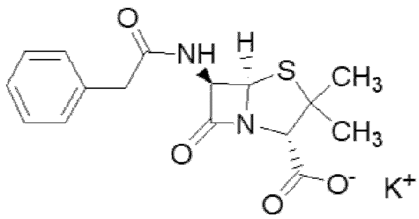 |
| Carbenicillin sodium | 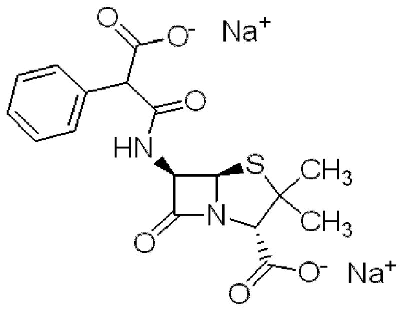 |

(continued)

| Penicillin compounds | Molecular structural formula |
|---|---|
| Oxacillin sodium | 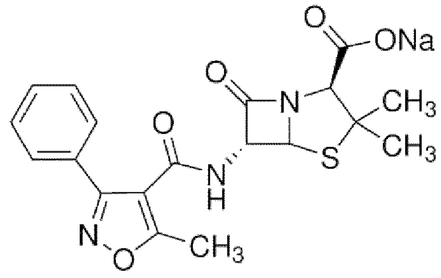 |
| Ampicillin sodium | 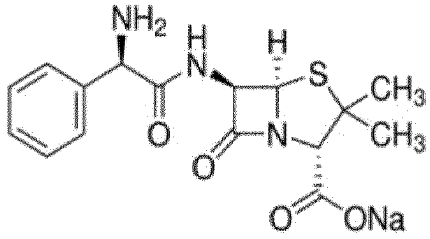 |
| Ampicillin | 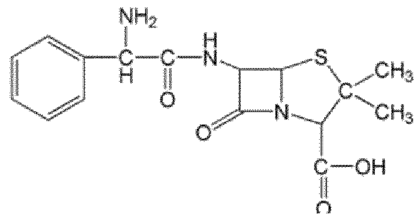 |

In order to further promote the nucleation and growth of hydrate, thereby further shortening the induction time of hydrate, further increasing the growth rate and gas storage capacity of hydrate, and further increasing the gas recovery rate during the decomposition process of hydrate, the surfactant is consisting of a zwitterionic surfactant and a nonionic surfactant as further described in claim 1.

In reference embodiments, component A is selected from the cephalosporin compounds, the surfactant is consisting of a Tween series polyol type nonionic surfactant and an alkylphenol polyoxyethylene ether nonionic surfactant. The inventors of the present disclosure have found that when the surfactant is consisting of a Tween series polyol type nonionic surfactant and an alkylphenol polyoxyethylene ether nonionic surfactant, it can match with the cephalosporin compounds, significantly improve the growth rate of hydrate and gas storage capacity, and further improve the gas recovery rate during decomposition process of hydrate, and further shorten the induction time of hydrate.

In reference embodiments, the component A is selected from the cephalosporin compounds, a mass ratio between the Tween series polyol type nonionic surfactant and the alkylphenol polyoxyethylene ether nonionic surfactant is (0.1-10):1, the mass ratio may be, for example, a ratio of 1 relative to the following numerical values: 0.1, 0.5, 1.0, 1.5, 2.0, 2.5, 3.0, 3.5, 4.0, 4.5, 5.0, 5.5, 6.0, 6.5, 7.0, 7.5, 8.0, 8.5, 9.0, 9.5 or 10, and a ratio between 1 and a random value between any two numerical values of 0.1-10; the mass ratio is preferably (0.2-5):1, further preferably (0.5-2):1, so as to further shorten the induction time, further improve the generation rate and gas storage capacity of hydrate, and can further reduce the amount of air bubbles generated during the decomposition process of hydrate. More preferably, the Tween series polyol type nonionic surfactant is one or more selected from the group consisting of Tween 20, Tween 40, Tween 60, Tween 65, Tween 80, and Tween 85 polyol type nonionic surfactant; further preferably, the alkylphenol polyoxyethylene ether nonionic surfactant is represented by the general

Formula 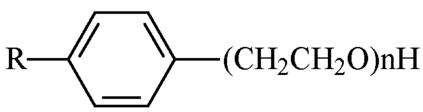 ,

wherein R is selected from $C_8$-$C_{18}$ alkyl group, n is the addition number of ethylene oxide, and n is an integer selected from 6-30.

According to the present disclosure, the component A is selected from the penicillin compounds, and the surfactant is consisting of a betaine type zwitterionic surfactant and a fatty alcohol polyoxyethylene ether non-ionic surfactant, it can further increase the generation rate and gas storage capacity of hydrate, and further shorten the induction time, and further decreases the amount of air bubbles generated during the decomposition process of hydrate. A mass ratio of the betaine type zwitterionic surfactants and the fatty alcohol polyoxyethylene ether non-ionic surfactants is (0.1-10):1, the mass ratio may be, for example, a ratio of 1 relative to the following numerical values: 1: 0.1, 0.5, 1.0, 1.5, 2.0, 2.5, 3.0, 3.5, 4.0, 4.5, 5.0, 5.5, 6.0, 6.5, 7.0, 7.5, 8.0, 8.5, 9.0, 9.5 or 10, and a ratio between 1 and a random value between any two numerical values of 0.1-10; more preferably (0.2-5):1, further preferably (0.5-2):1.

According to the present disclosure, there are many options for the betaine type zwitterionic surfactants. Preferably, the betaine type zwitterionic surfactant is one or more selected from the group consisting of alkyl betaines, alkyl amide betaines, sulfobetaines, sulfate betaines and phosphate betaines. The general structural formula of the betaine type zwitterionic surfactants is shown in the

Formula 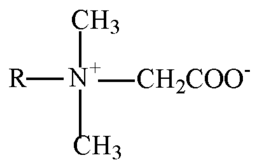 ,

wherein when the group R is substituted by an alkyl group, an alkyl betaine can be obtained, typically an alkyl group of $C_{12}$-$C_{14}$. A modified form of alkyl betaines, such as alkyl amide betaines, can be obtained if the other functional groups (e.g., ether linkages, hydroxyl groups, amide groups) are introduced into the alkyl chain of R. The zwitterionic surfactants such as sulfobetaines, sulfate betaines, and phosphate betaines, can be obtained if an acetate group is substituted by another group.

According to the present disclosure, the general structural formula of the fatty alcohol polyoxyethylene ether nonionic surfactants is $RO(CH_2CH_2O)_nH$; wherein the group R is an alkyl group, n is the addition number of ethylene oxide, and n is an integer selected from 2-30.

The inventors of the present disclosure have proposed the following speculation about the mechanism that no air bubble is generated during the promotion and decomposition processes of the hydrate promoter in regard to the hydrate.

(1) Before the formation of hydrate, since core component comprises the component A (e.g., a penicillin-type compound) of a substance having a group represented by the following Formula I

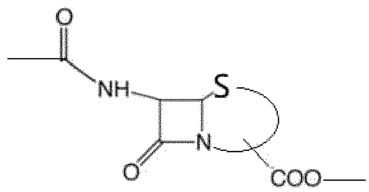

I

and the molecular structural formula of the surfactant component comprises both a hydrophilic group and a hydrophobic group, thus the hydrophilic groups in the core component and the surfactant molecules stretch into the liquid phase, while the hydrophobic groups are toward the gas phase, and the lipophilic structure of the hydrophobic groups is capable of adsorbing an organic gas (e.g., methane) so that the core component has a certain function of increasing dissolution of the gas, resulting in an increased concentration of the gas at the gas-liquid interface.

(2) Initial formation of hydrate particles. Given that the wall surface of a reactor is conducive to heat transfer, thus the gas-liquid-reactor wall is the initial formation point of hydrate; unlike the uniform nucleation and growth of the pure water systems, the hydrate is rapidly nucleated and grown by the action of the above-mentioned hydrate promoter contained therein; after the presence of the hydrate particles in the hydrate of the system, the core component transfers the gas-liquid interface to the surface of the hydrate particles, the hydrophilic groups and lipophilic groups contained therein are used to induce the continuous and rapid growth of the hydrate particles, and the formed hydrate has a porous structure, and the unreacted aqueous phase is rapidly sucked and filled into the hydrate pores through the capillary force, causing the central part remote from the wall surface to collapse, thereby exhibiting the wall-climbing phenomenon of the hydrate on the wall surface.

(3) Because the hydrate having a porous structure on the wall surface has a larger contact space with the gas phase, and it is beneficial to heat transfer through the reactor wall (since the hydrate reaction is an exothermic reaction, and the rapid removal of heat facilitates the continuous proceeding of the reaction), thus the hydrate exhibits a form that grows rapidly along the reactor wall under the action of the hydrate promoter, until an end of the reaction; therefore, the hydrate promoter of the present disclosure is still able to promote the formation of hydrate with a high generation rate and gas storage capacity under a circumstance without generating air bubbles.

(4) Decomposition and recovery of hydrate. After raising the temperature to the phase equilibrium temperature corresponding to the pressure of the system, the storage state of hydrate is destabilized, and hydrate is gradually decomposed from the reactor walls due to the advantage that the reactor walls are conducive to heat transfer, the decomposed gas phase diffuses to the gas phase space in the system, the decomposed aqueous phase gradually accumulates to the bottom of the reactor due to the action of gravity, given that the hydrate promoter has a limited reduction on the surface tension of an aqueous phase, the aqueous phase in the meanwhile still has a high surface tension, which is close to that of the pure aqueous phase, thereby suppressing generation of air bubbles, the significant generation of air bubbles is not visible in the disturbance during the process of decomposition and gas release, such that the decomposition efficiency and gas recovery rate of hydrate can be improved.

In a preferred embodiment of the present disclosure, the hydrate promoter further comprises an adjuvant selected from a polysaccharide gum and/or a water-soluble inorganic salt.

According to the present disclosure, in order to further promote the nucleation and growth of hydrate, thereby further shortening the induction time of hydrate and further increasing the growth rate of hydrate, and further improving the gas recovery rate during decomposition process of hydrate, a mass ratio of the component A to the adjuvant is 1: (0.1-5); that is, a mass ratio of the component A, the surfactant and the adjuvant in the hydrate promoter is 1: (0.1-10): (0.1-5); preferably 1: (0.1-5): (0.1-5).

In reference embodiments, the component A is a cephalosporin compound, the adjuvant is preferably a polysaccharide gum. The polysaccharide gum refers to highly linear, chain-like macromolecules with or without branched chain, the branched chain is short, and uniformly or non-uniformly distributed on the chain backbone. A sugar base forming the polysaccharides may be divided into several groups, it may be neutral, alkaline or acidic sugar base, with or without charged groups in the sugar base. The chain twisting, empty groups of branched chain, electrostatic repulsion and bond tension reduce the freedom degree of conformational change of the polysaccharide molecules, a part of which has higher conformations such as spirals and oval boxes, thus the polysaccharide molecules are less liable to crystallize and are generally present in an amorphous state. Due to the high spreading degree of the molecules, many hydrophilic groups are dissociated and exposed, the molecules have high hydrophilicity. Such polysaccharides may be collectively referred to as polysaccharide gums. In the present disclosure, polysaccharide gums can promote stability of hydrate, thereby promoting formation of the hydrate.

The polysaccharide gum may be at least one selected from the group consisting of locust bean gum, guar gum, tara gum, fenugreek gum, flax seed gum, algin gum, xanthan gum, gum arabic and chitosan; further preferably xanthan gum. Xanthan gum is a single cell polysaccharide produced by fermentation of the Pseudomonas, it is prepared by using the carbohydrate contained in Brassica oleracea Xanthomonas as the main raw material, which is treated with the aerobic fermentation bioengineering technology to cleave 1,6-glycosidic bonds, open the branched chain, and synthesize a linear chain at 1,4-bonds to form an acidic exopolysaccharide, its molecular structural formula is shown in Formula IV, and has a weight-average molecular weight of 2-50$\times 10^6$. Due to its macromolecular specific structural and colloidal characteristics, the xanthan gum has various functions, can be used as an emulsifier, a stabilizer, and a wetting agent, and has desirable biodegradability.

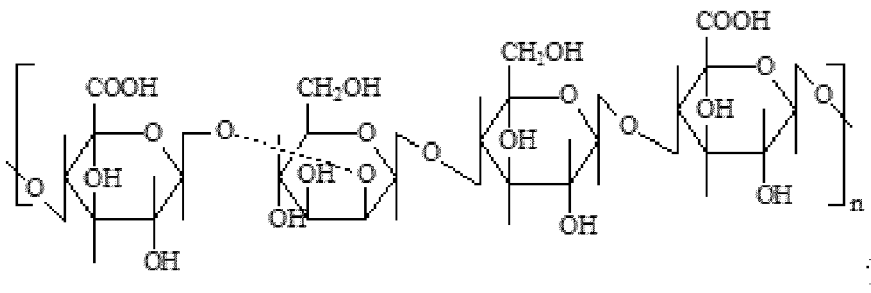

Formula IV.

For the sake of further improving the gas recovery rate during the decomposition process of hydrate, it is arranged in a reference embodiment of the present disclosure that a mass ratio of the cephalosporin compound, the polysaccharide gum and the surfactant is 1: (0.1-1): (0.5-5); further preferably, the mass ratio of the cephalosporin compound, the

polysaccharide gum and the surfactant is 1: (0.3-0.6): (1-5).

In a preferred embodiment of the present disclosure, in order to further promote the nucleation and growth of hydrate, thereby further shortening the induction time of hydrate and further increasing the growth rate of hydrate, and further improving the gas recovery rate during the decomposition process of hydrate, it is preferable that the component A is the penicillin compound, and the adjuvant is selected from the water-soluble inorganic salts; further preferably, a mass ratio of the penicillin compound, the surfactant and the water-soluble inorganic salt is 1: (0.1-5): (0.1-5), more preferably 1: (0.1-1): (0.1-5); further preferably, the water-soluble inorganic salt is at least one selected from the group consisting of $Na_2CO_3$, $K_2CO_3$, NaCl, KCl and $MgCl_2$.

In the oil and gas hydrate slurries, the water phase in the system is prone to react with the gas to form a solid hydrate, which causes blockage of the pipeline. The problem has been the hot and important focus of researchers at home and abroad in recent years concerning how to suppress or control the plugging of pipeline by gas hydrate in oil and gas production and transportation pipeline. A hydrate polymerization inhibitors are new inhibitors used with low dose, which can reduce the cohesive force between the hydrate particles and allow the hydrate particles to be conveyed in a form of hydrate slurry without aggregation and blockage. The currently used the hydrate polymerization inhibitors only serve to inhibit the cohesive force between the particles after formation of hydrate, but those skilled in the art do not consider the function of the hydrate polymerization inhibitors on the formation of hydrate, thus the hydrate polymerization inhibitors have a single functionality. The hydrate polymerization inhibitors in the prior art, such as those in the Chinese patent applications ZL201110096579.2, CN02115154.7A and ZL201510501017.X, while inhibiting the aggregation of hydrate, the hydrate polymerization inhibitors have not acceleration effect on the nucleation and growth of hydrate, or impose an inhibitive effect on the nucleation and growth rate of hydrate. It can be seen that the dual functions of simultaneously increasing the growth rate of hydrate and the preventing aggregation between the hydrate particles cannot be accomplished in the prior art.

The inventors of the present disclosure have discovered that the addition of an anti-agglomerant in a hydrate promoter, wherein the molecular structure of the anti-agglomerant comprises carbonyl groups and/or hydroxyl groups and a plurality of cyclic structures, the anti-agglomerant not only can promote the generation of hydrate, increase the generation rate and gas storage capacity of hydrate, but also can inhibit the aggregation of hydrate. The reasons may be that the carbonyl groups and/or hydroxyl groups in the molecular structure of the anti-agglomerant have desirable hydrophilic properties, and a plurality of cyclic structures in the molecular structure have lipophilic properties, such a structure allows the anti-agglomerant to be easily attached to the oil-water interface, so that the anti-agglomerant adheres to the surface of the hydrate particles, and by reducing the cohesive force between the hydrate particles, the hydrate particles per se can be in a dispersed state without aggregation, serving the purpose of preventing agglomeration of the hydrate particles, thereby increasing the generation rate of hydrate. Along with the rapid growth of hydrate, the anti-agglomerant can be easily adhered to the surface of the hydrate particles because of its hydrophilic groups, while the hydrophobic groups disperse and isolate the hydrate particles, serving the purpose of preventing agglomeration of the hydrate particles without affecting the rapid formation of hydrate, furthermore, the anti-agglomerant not only does not inhibit the formation of hydrate, but serves the purpose of preventing the accumulation of the hydrate particles, but also increases the stability of hydrate, and in cooperation with other active ingredients in the hydrate promoter, it can increase the nucleation and growth rate of hydrate, and improve the gas storage capacity, thus the anti-agglomerant provides dual functions of promoting the nucleation and growth of hydrate and preventing the agglomeration of the hydrate particles. The present disclosure has a favorable application prospect in the aspects of controlling the hydrate blockage and promoting the gas storage of the hydrate slurry.

Conventional amphiphilic molecules containing hydrophobic groups and hydrophilic groups either do not attach to the surface of the hydrate particles and the hydrophobic groups disperse and isolate the hydrate particles, such that the amphiphilic molecules do not serve the purpose of preventing aggregation of hydrate, or inhibit the formation of hydrate and reduce the generation rate of hydrate formation, the molecular structure of the anti-agglomerant preferably contains carbonyl groups and/or hydroxyl groups and a plurality of cyclic structures. In order to further improve the performance of the hydrate polymerization inhibitors for preventing agglomeration of hydrate, the anti-agglomerant preferably contains 3-4 multi-member rings; further preferably, the multi-member ring is a five-member ring or a six-member ring; still further preferably, the multi-member ring is selected from phenyl or a group containing at least one of a C-C unsaturated bond and an ester group.

The total number of carbonyl groups and hydroxyl groups is preferably 2-4, and the carbonyl group can be either carboxyl COOH or ester group COOR.

Each of the anti-agglomerants having the above molecular structure can fulfill the purpose the present disclosure in cooperation with the component A and the surfactant, furthermore, in order to enhance the performance for suppressing the aggregation of hydrate and further enhance the generation rate and the gas storage capacity of the hydrate; preferably, the anti-agglomerant is a statin organic; and further preferably, at least one selected from the group consisting of pravastatin, lovastatin, simvastatin and atorvastatin. The molecular structural formula of some statin organics are illustrated in Table 3.

Table 3. Molecular structural formula of statin organic compounds

| | | |
|---|---|---|
| 1 | Atorvastatin | 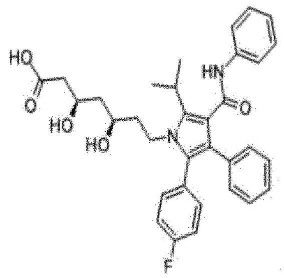 |
| 2 | Simvastatin | 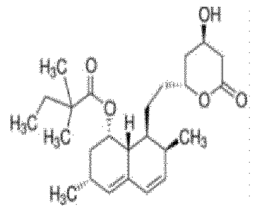 |
| 3 | Lovastatin | 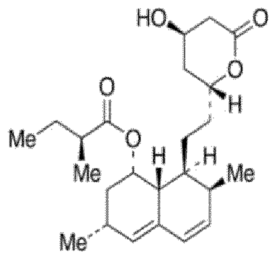 |

According to the present disclosure, the content of the anti-agglomerant can be adjusted within a wide range. Preferably, the anti-agglomerant is contained in an amount of 1-10 parts by mass, more preferably 3-6 parts by mass, relative to a total amount of 1 part by mass of the component A and the surfactant and optionally the adjuvant contained in the hydrate promoter. The content of anti-agglomerant may be, for example, 3 parts by mass, 4 parts by mass, 5 parts by mass, 6 parts by mass, or an arbitrary value between any two numerical values.

The inventors of the present disclosure have discovered that current technology for storing natural gas with hydration method requires continuous mechanical reinforcement during hydrate formation, particularly during the initial stage of hydrate formation, and it requires a mechanical agitation unit disposed in the device, thus imposes a high requirement on the device. In order to further overcome the technical problems in the prior art that requires continuous mechanical reinforcement during hydrate formation, particularly during the initial stage of hydrate formation, and it requires a mechanical agitation unit disposed in the device thereby imposing a high requirement on the device, the hydrate promoter further comprises an effervescent agent, which is capable of voluntarily releasing gas in water. In this way, the hydration promoter per se is capable of generating air bubbles to "effervesce", the air bubbles increase the contact area of the active ingredient with the gas to replace or cooperate with mechanical reinforcement, thereby providing motive force to the process of generating hydrate, particularly during the nucleation and initial growth stage of hydrate, thereby promoting continuous formation of hydrate. Therefore, the hydration promoter can produce a high generation rate of hydrate during the hydrate formation process, and can reduce the investment cost of the device and save more energy consumption.

In accordance with the present disclosure, a mass ratio of the active ingredient (e.g., component A) and the effervescent agent can be flexibly adjusted; in order to provide more motive force to the nucleation and initial growth stage of hydrate, even in the circumstance of without mechanical reinforcement or the generation is performed simultaneously with the mechanical reinforcement, the composition can further increase the generation rate of hydrate. Preferably, the effervescent agent is used in an amount of 0.01-0.5 parts by mass, more preferably 0.02-0.3 parts by mass, further preferably 0.02-0.2 parts by mass, relative to a total amount of 1 part by mass of the component A and the surfactant and optionally the adjuvant contained in the hydrate promoter.

The present disclosure does not impose particular limitation to the effervescent agent, the present disclosure can be implemented as long as the effervescent agent releases gas in water. Preferably, the effervescent agent comprises an acid source and an alkali source which are separately stored; preferably, a mass ratio of the acid source and the alkali source is 1: (0.5-2), more preferably 1: (0.8-1.2).

It is further preferred that the acid source is at least one selected from the group consisting of citric acid, malic acid, boric acid, tartaric acid and fumaric acid, and the alkali source is selected from a basic carbonate and/or a basic

bicarbonate, still further preferably at least one selected from the group consisting of sodium bicarbonate, sodium carbonate, potassium carbonate and potassium bicarbonate. In this way, the organic acid source reacts with the basic carbonate or bicarbonate salt to release carbon dioxide, produces a large number of air bubbles in the hydrate formation system, increases the contact surface of the hydrate promoter with the gas, provides motive force to the process of generating hydrate, particularly during the nucleation and initial growth stage of hydrate, thereby promoting continuous formation of hydrate.

The effervescent agent described above is commercially available and can also be prepared with the existing methods.

The preparation method of the hydrate promoter in the present disclosure is simple, the raw materials may be mixed, or the raw materials are separately stored and respectively added according to the matched ratios in use. Preferably, the effervescent agent is stored separately from the other components of the hydrate promoter, so that in use, the effervescent agent and the other components of the hydrate promoter are added into the aqueous system in a stepwise manner, it is preferred that the effervescent agent is added into the aqueous system after the addition of the other components of the hydrate promoter, such that the air bubbles voluntarily released by the effervescent agent in water can cause a stronger agitation in the aqueous system, thereby further increasing the generation rate of hydrate.

In a second aspect, the present disclosure provides a method for preparing a hydrate, comprising contacting a gas in an aqueous system with the hydrate promoter of the first aspect under the hydrate formation conditions.

The hydrate promoter provided by the present disclosure is a decomposition bubble-free hydrate promoter, the preparation method of the hydrate can effectively promotes rapid growth of hydrate, the produced hydrate has a high gas storage capacity, and the air bubbles are not generated during the decomposition process of hydrate, the recovery efficiency of gas generated during the decomposition process is high.

According to the present disclosure, the hydrate formation conditions may be the conventional conditions in the art. Preferably, the hydrate formation conditions comprise: a temperature of -20°C to 50°C; a pressure of 0.1-20MPa.

According to the present disclosure, the used amount of the hydrate promoter may be adjusted within a wide range, a mass ratio of the total mass of the component A and the surfactant and optionally added adjuvant relative to water is preferably (0.1-10):100; more preferably (0.1-5):100.

In accordance with the present disclosure, it is preferred when the effervescent agent is included in the hydrate promoter, the other components of the hydrate promoter other than the effervescent agent are initially added into the aqueous system, the effervescent agent is subsequently added into the aqueous system when the conditions of the aqueous system satisfy the formation conditions of hydrate. This allows the components of the hydrate promoter to be added in a stepwise manner, which facilitates mixing of water with the ingredients of hydrate promoter other than the effervescent agent, improves the generation rate of hydrate, and prevents the effervescent agent from effervescing in advance; if the effervescent agent is effervescent when the conditions of the aqueous system satisfy the formation conditions of hydrate, the air bubbles voluntarily released by the effervescent agent in water can cause a stronger agitation in the aqueous system, thereby further increasing the generation rate of hydrate under the hydrate formation conditions.

Unless otherwise specified in the present disclosure, the pressure refers to a gauge pressure.

In a third aspect, the present disclosure provides a hydrate prepared with the preparation method of the second aspect.

In a fourth aspect, the present disclosure provides a hydrate comprising the hydrate promoter of the first aspect.

The hydrate provided by the present disclosure is a decomposition bubble-free hydrate, the hydrate also has a high gas storage capacity, the air bubbles are not generated during the decomposition process of hydrate, the recovery efficiency of gas generated during the decomposition process is high.

In a fifth aspect (not separately claimed), the present disclosure provides a hydrate-based storage and transportation method, the method comprises a hydrate production process, a hydrate storage and transportation process and a gas release process, wherein:

(1) the hydrate production process serves to obtain hydrate by dispersing the aforementioned hydrate promoter in an aqueous phase, and bringing a gas into contact with an aqueous system in which the hydrate promoter is dispersed under hydrate formation conditions, so as to obtain a hydrant;
(2) the hydrate storage and transportation process stores and transports the hydrate produced by the hydrate production process under hydrate self-protecting conditions;
(3) the gas release process releases the gas therein by decomposing the hydrate subjected to the hydrate storage and transportation process under hydrate decomposition conditions.

The gas of the present disclosure may be a single gas to be stored and transported, such as methane, ethane, propane, carbon dioxide and hydrogen; or a mixture of gases, such as natural gas, an associated gas in an oil production process, and an associated gas in a natural gas production process. The aqueous phase may be a pure aqueous phase or the oil-water phases. The oil phase of the oil-water phases may be a conventional petroleum product oil such as gasoline,

kerosene and fuel oil. The water-gas ratio may be a conventional ratio in the art, it is preferred that an excessive gas is filled according to a relationship of up to 185 cubic meters of gas stored in 1 cubic meter of water during the formation process of hydrate.

In a preferred embodiment of the present disclosure, the hydrate formation conditions comprise: a temperature of -20°C to 50°C; a pressure of 0.1-20MPa; preferably a temperature of -10°C to 20°C; a pressure of 1-15MPa.

Preferably, the temperature of the hydrate self-protective effect is 253.15K-270.15K, and the pressure range is 0-1.0MPa.

Unless otherwise specified in the present disclosure, the pressure refers to a gauge pressure, for example, when the pressure is 0MPa, which means an atmospheric pressure condition.

In order to improve the safety of the hydrate storage and transportation, it is preferred that the hydrate storage and transportation process comprises monitoring the parameters of the hydrate-containing system during the storage and transportation process, and maintaining the hydrate within the target temperature and pressure ranges of storage and transportation by adjusting the temperature of the hydrate system or releasing the gas; when the pressure of the hydrate system is higher than the target value during the hydrate storage and transportation process, the parameters of the hydrate-containing system in the storage and transportation unit can be maintained within the target ranges of storage and transportation by means of releasing a portion of gas or lowering the system temperature.

The transportation mode of the hydrate may be traction-handling by a power equipment. The power equipment may be the existing transport tool such as a vehicle and a boat. Preferably, the hydrate-containing system in the hydrate storage and transportation process has a temperature of 243.15-270.15K and a pressure not higher than 0.5 MPa.

The release of gas from the decomposition of hydrate may be achieved in various manners. Preferably, the gas release process decomposes the hydrate by raising the temperature and/or decreasing the pressure to release the gas; in order to improve the decomposition efficiency, the temperature of the gas release process is preferably within a range of 273.15-323.15K.

The temperature rise may be performed according to a target temperature by using a conventional heating means, and decreasing the pressure may be performed by using a conventional pressure reduction mode such as opening a valve, the relevant contents will not be repeated herein.

In the above technical scheme, the monitoring of pressure and temperature may be performed by using a conventional configuration mode in the art, for example, the pressure monitoring may be performed by using a pressure sensor, the temperature monitoring may be implemented by a temperature sensor. In order to maintain stability of the reaction gas pressure during the addition process of an effervescent agent, it is preferred that a medicine storage cartridge is provided in the reactor, the effervescent agent is placed in the medicine storage cartridge in advance, when the conditions of the system satisfy the reaction conditions, the effervescent agent in the medicine storage cartridge is poured into the reactor to contact with the water phase. The connection of the medicine storage cartridge with the reactor may be performed with multiple choices, for example, the medicine storage cartridge is connected to the reactor by a shaft and the connection shaft extends to the outside of the reactor, an overturn of the medicine storage cartridge is controlled by the connection shaft; or a valve is provided at the bottom of the medicine storage cartridge, the opening of the bottom of the medicine storage cartridge is controlled from the outside; each pertains to the conventional configuration in the art. In the examples described below, a valve is provided at the bottom of the medicine storage cartridge.

Since the gas-liquid surface tension is an important parameter for reflecting the generation of air bubbles and stability of the air bubble structure, the lower is the gas-liquid surface tension, it means that air bubbles are more likely to be generated under an external disturbance, and the stability of air bubbles is stronger; given that the aforementioned hydrate promoter system does not generate air bubbles during the decomposition process of hydrate, the effect of the hydrate promoter of the present disclosure on the surface tension of an aqueous phase is limited, preferably, the surface tension of the aqueous phase containing the hydrate promoter is within a range of 50mN/m - 68mN/m under the conditions comprising a temperature of 298.15K, an atmospheric pressure, and the desired concentration range.

The present disclosure also provides a hydrate promoter-containing hydrate decomposition gas recovery and gas recovery rate calculation method (not separately claimed), the method comprises the following steps:

(1) after the formation of hydrate in the reaction system is complete, the system temperature is rapidly increased above the hydrate phase equilibrium temperature corresponding to the system pressure;

(2) after the pressure of the system to be decomposed is stabilized and maintained for 1.0 hour, the gas in the system following the decomposition is metered on-line by a gas flow meter, if the system following the decomposition has a foam shape or the system changes to a foam shape during the decomposition process, the gas discharge is stopped; if the system is still foamed shaped after 30min, it is deemed that the gas cannot be effectively recovered following the state;

(3) calculating an actual gas storage capacity of hydrate based on the change in gas pressure and the reaction system parameters during the generation process of hydrate, the total amount of gas flow recorded by the gas flow meter following decomposition is the actual recovered gas amount, and a ratio of the actual recovered gas amount to the

actual gas storage capacity is the gas recovery rate.

The present disclosure will be described below in detail with reference to examples. In the following examples, the performance evaluation was performed by using an autoclave and the specific experimental procedure was as follows.

(1) The whole experimental system was washed, the test solution (10 mL) containing the hydrate promoter is prepared and placed in the autoclave, the system was vacuumized and introduced with the experimental gas and replaced with the experimental gas for 3 times or more; when the hydrate promoter comprised an effervescent agent, the effervescent agent was added separately.
(2) The system temperature was set to the experimental temperature, when the temperature in the autoclave reached the preset value and kept stable for 5 hours, a certain amount of experimental gas was introduced to bring the system to reach a dissolution equilibrium (the pressure of introduced gas was less than the corresponding hydrate equilibrium pressure at the temperature).
(3) The test gas was introduced to a test pressure, an air intake valve was opened, a stirrer was powered, the stirring speed was constant throughout the whole experimental process, the timing was started, the macro morphological changes in the system were observed and recorded on-line with a video recorder, the system temperature, pressure and reaction time were automatically recorded by using a computer data automatic acquisition system.
(4) When the white hydrate particles were present in the system, the time was recorded as an induction time of hydrant.
(5) The experiment was continued, the evolution of the macrostructure of the hydrate in the system after the appearance of hydrate particles was observed in real time; meanwhile, the system pressure was recorded for different periods of reaction, starting from the induction time.
(6) Along with the continuous formation of the hydrate, when the system pressure was stabilized and maintained for 2.0h, the system temperature was adjusted to 298.15K, the macroscopic morphological evolution of the decomposition process of hydrate in the autoclave (mainly observing whether the air bubbles were generated) was observed in real time; after the gas hydrate in the autoclave was completely decomposed, the vent valve was opened, the decomposed gas was metered on-line by the gas flow meter; if the system following the decomposition had a foam shape or the system changed to a foam shape during the decomposition process, the gas discharge was stopped; if the system was still foamed shaped after 30min, it was deemed that the gas cannot be effectively recovered following the state, the experiment was stopped, the surface tension of the solution at 298.15K following reaction was tested by the surface tension instrument, and the next group of experiments was restarted.

The gas storage capacity of hydrate and the gas recovery rate were calculated with the following method: the amount of consumed gas for hydrate formation was:

$$n_c = n_0 - n_t \quad (1);$$

wherein $n_c$ denoted the amount of consumed gas required for hydration formation from the moment when hydration particles began to occur to the time t, $n_0$ denoted the molar weight of gas in the corresponding system when the experiment proceeded to an induction time, and $n_t$ denoted the molar weight of gas in the corresponding system when the experiment proceeded to the time t.

$$n_c = (P_0 Z_0 - P_t Z_t)\frac{V_g}{RT} \quad (2);$$

Formula (1) can also be written as Formula (2) based on the gas state equation, wherein $P_0$ and $P_t$ denoted the system pressures when the experiment was carried out to the induction time of and the time t, $Z_0$ and $Z_t$ were the respective gas compression factors of the corresponding state (calculated from the Peng-Robinson state equation), $V_g$ denoted the volume of gas phase space in the system, R denoted a gas constant, and T denoted the experimental temperature.

$$V_c = \frac{n_c \times 22.4 \times 1000}{1.25 \times V_l} \quad (3);$$

wherein $V_c$ in Formula (3) denoted the gas storage capacity per unit volume of hydrate in the system, $V_l$ denoted the initial liquid phase volume, because the volume expanded about 1.25 times after conversion of the aqueous phase to hydrate, thus the 1.25 times of the initial liquid phase volume was regarded as the volume of the final hydrate in Formula (3).

$$R\% = \frac{V_c}{V_d} \times 100\% \quad (4);$$

in Formula (4), R denoted the gas recovery rate after the decomposition of hydration, and $V_d$ denoted the gas volume obtained through the gas flow meter during the process of decomposition and gas discharge.

The gas used in the Comparative Examples and Examples described below was methane gas having a purity of 99.99%.

The present disclosure will be described below in detail with reference to examples.

Example 1

The present disclosure provided a hydrate promoter, which was obtained by mixing penicillin sodium, dodecyldimethyl betaine, $C_{12}H_{25}O(CH_2CH_2O)_5H$ and $Na_2CO_3$ in a mass ratio of 1:0.05:0.05:0.5.

The example was evaluated by using an autoclave, 10 mL of deionized water was prepared and added into the autoclave along with the hydrate promoter in an amount of 0.5% by mass of water in the system, the experimental pressure was 7.0 MPa, and the experimental temperature was 276.2K, it was discovered by the autoclave that the induction time for hydrate was 1.5 min.

Started from the appearance of the hydrate particles in the system, the system pressures were 4334kPa, 3656.82kPa and 3573.66kPa, respectively when the reaction proceeded to 30 min, 60 min and 120 min, it was calculated according to Formula (1) - Formula (3) that the corresponding gas storage capacities were 136.09 V/V, 168.13 V/V and 171.09 V/V, respectively when the reaction proceeded to 30 min, 60 min and 120 min.

The system temperature was raised to 298.15K after completion of hydrate formation, it was observed through the autoclave that no significant air bubbles appeared in the autoclave during the decomposition process of hydrate; after completion of the decomposition, the actual recovery volume of gas after decomposition was measured by an exhaust valve and a gas flow meter, the gas recovery rate was calculated to be 97% according to Formula (4). In addition, the surface tension (at a temperature of 298.15K) of the liquid phase in the autoclave after completion of the experiment was measured to be 68mN/m, as can be seen, the hydrate promoter in the example had small influence on the surface tension of the water phase, no significant air bubbles appeared during the decomposition process of hydrate, the gas recovery rate was high while ensuring a high gas storage capacity in the hydrate method.

Example 2

The present disclosure provided a hydrate promoter, which was obtained by mixing penicillin potassium, dodecyldimethyl betaine, $C_{12}H_{25}O(CH_2CH_2O)_5H$ and $K_2CO_3$ in a mass ratio of 1:0.05:0.05:0.5.

The example was evaluated by using an autoclave, 10 mL of deionized water was prepared and added into the autoclave along with the hydrate promoter in an amount of 0.5% by mass of water in the system, the experimental pressure was 7.0 MPa, and the experimental temperature was 276.2K, it was discovered by the autoclave that the induction time for hydrate was 1.75 min.

Started from the appearance of the hydrate particles in the system, the system pressures were 4617.76kPa, 3767.45kPa and 3534.75kPa, respectively when the reaction proceeded to 30 min, 60 min and 120 min, it was calculated according to Formula (1) - Formula (3) that the corresponding gas storage capacities were 117.72 V/V, 158.26 V/V and 170.06 V/V, respectively when the reaction proceeded to 30 min, 60 min and 120 min.

The system temperature was raised to 298.15K after completion of hydrate formation, it was observed through the autoclave that no significant air bubbles appeared in the autoclave during the decomposition process of hydrate; after completion of the decomposition, the actual recovery volume of gas after decomposition was measured by an exhaust valve and a gas flow meter, the gas recovery rate was calculated to be 96% according to Formula (4). In addition, the surface tension (at a temperature of 298.15K) of the liquid phase in the autoclave after completion of the experiment was measured to be 65mN/m, as can be seen, the hydrate promoter in the example had small influence on the surface tension of the water phase, no significant air bubbles appeared during the decomposition process of hydrate, the gas recovery rate was high while ensuring a high gas storage capacity in the hydrate method.

Example 3

The present disclosure provided a hydrate promoter, which was obtained by mixing carbenicillin sodium, dodecyldimethyl betaine, $C_{12}H_{25}O(CH_2CH_2O)_5H$ and $K_2CO_3$ in a mass ratio of 1:0.05:0.05:0.5.

The example was evaluated by using an autoclave, 10 mL of deionized water was prepared and added into the autoclave along with the hydrate promoter in an amount of 0.5% by mass of water in the system, the experimental pressure was 7.0 MPa, and the experimental temperature was 276.2K, it was discovered by the autoclave that the induction time for hydrate was 2.14 min.

Started from the appearance of the hydrate particles in the system, the system pressures were 4677.27kPa, 3928.05kPa and 3742.42kPa, respectively when the reaction proceeded to 30 min, 60 min and 120 min, it was calculated according to Formula (1) - Formula (3) that the corresponding gas storage capacities were 123.11 V/V, 159.01 V/V and 167.18 V/V, respectively when the reaction proceeded to 30 min, 60 min and 120 min.

The system temperature was raised to 298.15K after completion of hydrate formation, it was observed through the autoclave that no significant air bubbles appeared in the autoclave during the decomposition process of hydrate; after completion of the decomposition, the actual recovery volume of gas after decomposition was measured by an exhaust valve and a gas flow meter, the gas recovery rate was calculated to be 96% according to Formula(4). In addition, the surface tension (at a temperature of 298.15K) of the liquid phase in the autoclave after completion of the experiment was measured to be 66mN/m, as can be seen, the hydrate promoter in the example had small influence on the surface tension of the water phase, no significant air bubbles appeared during the decomposition process of hydrate, the gas recovery rate was high while ensuring a high gas storage capacity in the hydrate method.

Example 4

The present disclosure provided a hydrate promoter, which was obtained by mixing oxacillin sodium, dodecyldimethyl betaine, $C_{12}H_{25}O(CH_2CH_2O)_5H$ and $Na_2CO_3$ in a mass ratio of 1:0.05:0.05:0.5.

The example was evaluated by using an autoclave, 10 mL of deionized water was prepared and added into the autoclave along with the hydrate promoter in an amount of 0.5% by mass of water in the system, the experimental pressure was 7.0 MPa, and the experimental temperature was 276.2K, it was discovered by the autoclave that the induction time for hydrate was 2.1 min.

Started from the appearance of the hydrate particles in the system, the system pressures were 4580.21kPa, 3927.67kPa and 3717.86kPa, respectively when the reaction proceeded to 30 min, 60 min and 120 min, it was calculated according to Formula (1) - Formula (3) that the corresponding gas storage capacities were 123.48 V/V, 154.67 V/V and 164.51 V/V, respectively when the reaction proceeded to 30 min, 60 min and 120 min.

The system temperature was raised to 298.15K after completion of hydrate formation, it was observed through the autoclave that no significant air bubbles appeared in the autoclave during the decomposition process of hydrate; after completion of the decomposition, the actual recovery volume of gas after decomposition was measured by an exhaust valve and a gas flow meter, the gas recovery rate was calculated to be 97% according to Formula (4). In addition, the surface tension (at a temperature of 298.15K) of the liquid phase in the autoclave after completion of the experiment was measured to be 64mN/m, as can be seen, the hydrate promoter in the example had small influence on the surface tension of the water phase, no significant air bubbles appeared during the decomposition process of hydrate, the gas recovery rate was high while ensuring a high gas storage capacity in the hydrate method.

Example 5

The present disclosure provided a hydrate promoter, which was obtained by mixing penicillin sodium, dodecyldiethylhydroxyethyl betaine, $C_{12}H_{25}O(CH_2CH_2O)_7H$ and $Na_2CO_3$ in a mass ratio of 1:0.05:0.05:1.

The example was evaluated by using an autoclave, 10 mL of deionized water was prepared and added into the autoclave along with the hydrate promoter in an amount of 1% by mass of water in the system, the experimental pressure was 7.0 MPa, and the experimental temperature was 276.2K, it was discovered by the autoclave that the induction time for hydrate was 1.25 min.

Started from the appearance of the hydrate particles in the system, the system pressures were 4312.58kPa, 3636.22kPa and 3540.69kPa, respectively when the reaction proceeded to 30 min, 60 min and 120 min, it was calculated according to Formula (1) - Formula (3) that the corresponding gas storage capacities were 141.63 V/V, 173.56 V/V and 178.54 V/V, respectively when the reaction proceeded to 30 min, 60 min and 120 min.

The system temperature was raised to 298.15K after completion of hydrate formation, it was observed through the autoclave that no significant air bubbles appeared in the autoclave during the decomposition process of hydrate; after completion of the decomposition, the actual recovery volume of gas after decomposition was measured by an exhaust valve and a gas flow meter, the gas recovery rate was calculated to be 96% according to Formula (4). In addition, the

surface tension (at a temperature of 298.15K) of the liquid phase in the autoclave after completion of the experiment was measured to be 68mN/m, as can be seen, the hydrate promoter in the example had small influence on the surface tension of the water phase, no significant air bubbles appeared during the decomposition process of hydrate, the gas recovery rate was high while ensuring a high gas storage capacity in the hydrate method.

Example 6

The present disclosure provided a hydrate promoter, which was obtained by mixing penicillin sodium, dodecyldiethylhydroxyethyl betaine, $C_{12}H_{25}O(CH_2CH_2O)_7H$ and $Na_2CO_3$ in a mass ratio of 1:0.1:0.1:1.

The example was evaluated by using an autoclave, 10 mL of deionized water was prepared and added into the autoclave along with the hydrate promoter in an amount of 0.5% by mass of water in the system, the experimental pressure was 7.0 MPa, and the experimental temperature was 276.2K, it was discovered by the autoclave that the induction time for hydrate was 2.0 min.

Started from the appearance of the hydrate particles in the system, the system pressures were 4648.28kPa, 4034.87kPa and 3931.21kPa, respectively when the reaction proceeded to 30 min, 60 min and 120 min, it was calculated according to Formula (1) - Formula (3) that the corresponding gas storage capacities were 124.06 V/V, 153.51 V/V and 167.95 V/V, respectively when the reaction proceeded to 30 min, 60 min and 120 min.

The system temperature was raised to 298.15K after completion of hydrate formation, it was observed through the autoclave that no significant air bubbles appeared in the autoclave during the decomposition process of hydrate; after completion of the decomposition, the actual recovery volume of gas after decomposition was measured by an exhaust valve and a gas flow meter, the gas recovery rate was calculated to be 96% according to Formula (4). In addition, the surface tension (at a temperature of 298.15K) of the liquid phase in the autoclave after completion of the experiment was measured to be 63mN/m, as can be seen, the hydrate promoter in the example had small influence on the surface tension of the water phase, no significant air bubbles appeared during the decomposition process of hydrate, the gas recovery rate was high while ensuring a high gas storage capacity in the hydrate method.

Example 7

The present disclosure provided a hydrate promoter, which was obtained by mixing penicillin sodium, dodecyldiethylhydroxyethyl betaine, $C_{12}H_{25}O(CH_2CH_2O)_7H$ and $Na_2CO_3$ in a mass ratio of 1:0.1:0.2:1.

The example was evaluated by using an autoclave, 10 mL of deionized water was prepared and added into the autoclave along with the hydrate promoter in an amount of 0.5% by mass of water in the system, the experimental pressure was 7.0 MPa, and the experimental temperature was 276.2K, it was discovered by the autoclave that the induction time for hydrate was 2.5 min.

Started from the appearance of the hydrate particles in the system, the system pressures were 4771.52kPa, 3937.97kPa and 3723.96kPa, respectively when the reaction proceeded to 30 min, 60 min and 120 min, it was calculated according to Formula (1) - Formula (3) that the corresponding gas storage capacities were 117.48 V/V, 157.51 V/V and 167.55 V/V, respectively when the reaction proceeded to 30 min, 60 min and 120 min.

The system temperature was raised to 298.15K after completion of hydrate formation, it was observed through the autoclave that no significant air bubbles appeared in the autoclave during the decomposition process of hydrate; after completion of the decomposition, the actual recovery volume of gas after decomposition was measured by an exhaust valve and a gas flow meter, the gas recovery rate was calculated to be 97% according to Formula (4). In addition, the surface tension (at a temperature of 298.15K) of the liquid phase in the autoclave after completion of the experiment was measured to be 65mN/m, as can be seen, the hydrate promoter in the example had small influence on the surface tension of the water phase, no significant air bubbles appeared during the decomposition process of hydrate, the gas recovery rate was high while ensuring a high gas storage capacity in the hydrate method.

Reference Example 8

The hydrate promoter in the Example was obtained by merely mixing penicillin sodium and 2-butyloctylsulfate sodium (GC12S) in a mass ratio of 1:0.1.

The example was evaluated by using the method in Example 1, 10 mL of deionized water was prepared and added into an autoclave along with the hydrate promoter in an amount of 0.5% by mass of water in the system, the experimental pressure was 7.0 MPa, and the experimental temperature was 276.2K, it was discovered by the autoclave that the induction time for hydrate was 3.25 min.

Started from the appearance of the hydrate particles in the system, the system pressures were 4895.12kPa, 4205.62kPa and 3805.11kPa, respectively when the reaction proceeded to 30 min, 60 min and 120 min, it was calculated according to Formula (1) - Formula (3) that the corresponding gas storage capacities were 102.53 V/V, 138.56 V/V and

162.05 V/V, respectively when the reaction proceeded to 30 min, 60 min and 120 min.

The system temperature was raised to 298.15K after completion of hydrate formation, it was observed through the autoclave that no significant air bubbles appeared in the autoclave during the decomposition process of hydrate; after completion of the decomposition, the actual recovery volume of gas after decomposition was measured by an exhaust valve and a gas flow meter, the gas recovery rate was calculated to be 94% according to Formula (4). In addition, the surface tension (at a temperature of 298.15K) of the liquid phase in the autoclave after completion of the experiment was measured to be 60mN/m, as can be seen, the hydrate promoter in the example had small influence on the surface tension of the water phase, no significant air bubbles appeared during the decomposition process of hydrate, the gas recovery rate was high while ensuring a high gas storage capacity in the hydrate method.

Reference Example 9

The hydrate promoter in the Example was obtained by mixing penicillin sodium and dodecyldimethyl betaine in a mass ratio of 1:0.1.

The example was evaluated by using the method in Example 1, 10 mL of deionized water was prepared and added into an autoclave along with the hydrate promoter in an amount of 0.5% by mass of water in the system, the experimental pressure was 7.0 MPa, and the experimental temperature was 276.2K, it was discovered by the autoclave that the induction time for hydrate was 3.15 min.

Started from the appearance of the hydrate particles in the system, the system pressures were 4792.20kPa, 4135.87kPa and 3760.27kPa, respectively when the reaction proceeded to 30 min, 60 min and 120 min, it was calculated according to Formula (1) - Formula (3) that the corresponding gas storage capacities were 109.64 V/V, 142.51 V/V and 163.08 V/V, respectively when the reaction proceeded to 30 min, 60 min and 120 min.

The system temperature was raised to 298.15K after completion of hydrate formation, it was observed through the autoclave that no significant air bubbles appeared in the autoclave during the decomposition process of hydrate; after completion of the decomposition, the actual recovery volume of gas after decomposition was measured by an exhaust valve and a gas flow meter, the gas recovery rate was calculated to be 95% according to Formula (4). In addition, the surface tension (at a temperature of 298.15K) of the liquid phase in the autoclave after completion of the experiment was measured to be 62mN/m, as can be seen, the hydrate promoter in the example had small influence on the surface tension of the water phase, no significant air bubbles appeared during the decomposition process of hydrate, the gas recovery rate was high while ensuring a high gas storage capacity in the hydrate method.

Reference Example 10

The hydrate promoter in the Example was obtained by mixing penicillin sodium and $C_{12}H_{25}O(CH_2CH_2O)_5H$ in a mass ratio of 1:0.1.

The example was evaluated by using the method in Example 1, 10 mL of deionized water was prepared and added into an autoclave along with the hydrate promoter in an amount of 0.5% by mass of water in the system, the experimental pressure was 7.0 MPa, and the experimental temperature was 276.2K, it was discovered by the autoclave that the induction time for hydrate was 3.15 min.

Started from the appearance of the hydrate particles in the system, the system pressures were 4799.51kPa, 4123.45kPa and 3758.69kPa, respectively when the reaction proceeded to 30 min, 60 min and 120 min, it was calculated according to Formula (1) - Formula (3) that the corresponding gas storage capacities were 108.69 V/V, 141.22 V/V and 163.27 V/V, respectively when the reaction proceeded to 30 min, 60 min and 120 min.

The system temperature was raised to 298.15K after completion of hydrate formation, it was observed through the autoclave that no significant air bubbles appeared in the autoclave during the decomposition process of hydrate; after completion of the decomposition, the actual recovery volume of gas after decomposition was measured by an exhaust valve and a gas flow meter, the gas recovery rate was calculated to be 94% according to Formula (4). In addition, the surface tension (at a temperature of 298.15K) of the liquid phase in the autoclave after completion of the experiment was measured to be 60mN/m, as can be seen, the hydrate promoter in the example had small influence on the surface tension of the water phase, no significant air bubbles appeared during the decomposition process of hydrate, the gas recovery rate was high while ensuring a high gas storage capacity in the hydrate method.

Example 11

The hydrate promoter in the Example was obtained by mixing penicillin sodium, dodecyldimethyl betaine and $C_{12}H_{25}O(CH_2CH_2O)_5H$ in a mass ratio of 1:0.05:0.05.

The example was evaluated by using the method in Example 1, 10 mL of deionized water was prepared and added into an autoclave along with the hydrate promoter in an amount of 0.5% by mass of water in the system, the

experimental pressure was 7.0 MPa, and the experimental temperature was 276.2K, it was discovered by the autoclave that the induction time for hydrate was 2.75 min.

Started from the appearance of the hydrate particles in the system, the system pressures were 4710.25kPa, 4025.12kPa and 3755.62kPa, respectively when the reaction proceeded to 30 min, 60 min and 120 min, it was calculated according to Formula (1) - Formula (3) that the corresponding gas storage capacities were 115.33 V/V, 148.32 V/V and 164.15 V/V, respectively when the reaction proceeded to 30 min, 60 min and 120 min.

The system temperature was raised to 298.15K after completion of hydrate formation, it was observed through the autoclave that no significant air bubbles appeared in the autoclave during the decomposition process of hydrate; after completion of the decomposition, the actual recovery volume of gas after decomposition was measured by an exhaust valve and a gas flow meter, the gas recovery rate was calculated to be 95% according to Formula (4). In addition, the surface tension (at a temperature of 298.15K) of the liquid phase in the autoclave after completion of the experiment was measured to be 62mN/m, as can be seen, the hydrate promoter in the example had small influence on the surface tension of the water phase, no significant air bubbles appeared during the decomposition process of hydrate, the gas recovery rate was high while ensuring a high gas storage capacity in the hydrate method.

Reference Example 12

The hydrate promoter in the Example was obtained by mixing penicillin sodium, dodecylaminopropionate sodium and octylphenol polyoxyethylene ether TX-10 in a mass ratio of 1:0.05:0.05.

The example was evaluated by using the method in Example 1, 10 mL of deionized water was prepared and added into an autoclave along with the hydrate promoter in an amount of 0.5% by mass of water in the system, the experimental pressure was 7.0 MPa, and the experimental temperature was 276.2K, it was discovered by the autoclave that the induction time for hydrate was 2.85 min.

Started from the appearance of the hydrate particles in the system, the system pressures were 4702.68kPa, 4022.31kPa and 3760.41kPa, respectively when the reaction proceeded to 30 min, 60 min and 120 min, it was calculated according to Formula (1) - Formula (3) that the corresponding gas storage capacities were 113.25 V/V, 147.78 V/V and 163.58 V/V, respectively when the reaction proceeded to 30 min, 60 min and 120 min.

The system temperature was raised to 298.15K after completion of hydrate formation, it was observed through the autoclave that no significant air bubbles appeared in the autoclave during the decomposition process of hydrate; after completion of the decomposition, the actual recovery volume of gas after decomposition was measured by an exhaust valve and a gas flow meter, the gas recovery rate was calculated to be 95% according to Formula (4). In addition, the surface tension (at a temperature of 298.15K) of the liquid phase in the autoclave after completion of the experiment was measured to be 62mN/m, as can be seen, the hydrate promoter in the example had small influence on the surface tension of the water phase, no significant air bubbles appeared during the decomposition process of hydrate, the gas recovery rate was high while ensuring a high gas storage capacity in the hydrate method.

Example 13

The hydrate promoter was provided according to the method of Example 1, except that a mass ratio of penicillin sodium, dodecyldimethyl betaine, $C_{12}H_{25}O(CH_2CH_2O)_5H$ and $Na_2CO_3$ was 1:0.5:0.5:5.

Validation was carried out according to the method of Example 1, the induction time of hydrate was discovered to be 2.6min, the corresponding gas storage capacities were 115.85 V/V, 158.62 V/V and 167.22 V/V, respectively when the reaction proceeded to 30 min, 60 min and 120 min.

The gas recovery rate was 96%, and the surface tension (at a temperature of 298.15K) of the liquid phase in the autoclave after completion of the experiment was 65mN/m.

Reference Example 14

The present disclosure provided a hydrate promoter, which was obtained by mixing mono-hydrate cefchlorampin, xanthan gum, Tween 60 and $C_{15}H_{23}O(CH_2CH_2O)_7H$ in a mass ratio of 1:0.2:0.5:0.5.

The example was evaluated by using an autoclave, 10 mL of deionized water was prepared and added into the autoclave along with the hydrate promoter in an amount of 1.0% by mass of water in the system, the experimental pressure was 6.5 MPa, and the experimental temperature was 274.7K, it was discovered by the autoclave that the induction time for hydrate was 1.3 min.

Started from the appearance of the hydrate particles in the system, the system pressures were 3875kPa and 3125kPa, respectively when the reaction proceeded to 30 min and 60 min.

It was calculated according to Formula (1) - Formula (3) that the corresponding gas storage capacities were 135.95 V/V and 169.45 V/V, respectively when the reaction proceeded to 30 min and 60 min.

The system temperature was raised to 298.15K after completion of hydrate formation, it was observed through the autoclave that no significant air bubbles appeared in the autoclave during the decomposition process of hydrate; after completion of the decomposition, the actual recovery volume of gas after decomposition was measured by an exhaust valve and a gas flow meter, the gas recovery rate was calculated to be 96% according to Formula (4). In addition, the surface tension (at a temperature of 298.15K) of the liquid phase in the autoclave after completion of the experiment was measured to be 63mN/m, as can be seen, the hydrate promoter in the example had small influence on the surface tension of the water phase, no significant air bubbles appeared during the decomposition process of hydrate, the gas recovery rate was high while ensuring a high gas storage capacity in the hydrate method.

Reference Example 15

The present disclosure provided a hydrate promoter, which was obtained by mixing cefalexin, xanthan gum, Tween 60 and $C_{15}H_{23}O(CH_2CH_2O)_7H$ in a mass ratio of 1:0.2:0.5:0.5.

The example was evaluated by using an autoclave, 10 mL of deionized water was prepared and added into the autoclave along with the hydrate promoter in an amount of 1.0% by mass of water in the system, the experimental pressure was 6.5 MPa, and the experimental temperature was 274.7K, it was discovered by the autoclave that the induction time for hydrate was 1.5 min.

Started from the appearance of the hydrate particles in the system, the system pressures were 3956kPa and 3209kPa, respectively when the reaction proceeded to 30 min and 60 min.

It was calculated according to Formula (1) - Formula (3) that the corresponding gas storage capacities were 132.14 V/V and 166.76 V/V, respectively when the reaction proceeded to 30 min and 60 min.

The system temperature was raised to 298.15K after completion of hydrate formation, it was observed through the autoclave that no significant air bubbles appeared in the autoclave during the decomposition process of hydrate; after completion of the decomposition, the actual recovery volume of gas after decomposition was measured by an exhaust valve and a gas flow meter, the gas recovery rate was calculated to be 96% according to Formula (4). In addition, the surface tension (at a temperature of 298.15K) of the liquid phase in the autoclave after completion of the experiment was measured to be 64mN/m, as can be seen, the hydrate promoter in the example had small influence on the surface tension of the water phase, no significant air bubbles appeared during the decomposition process of hydrate, the gas recovery rate was high while ensuring a high gas storage capacity in the hydrate method.

Reference Example 16

The present disclosure provided a hydrate promoter, which was obtained by mixing cefotaxime, xanthan gum, Tween 60 and $C_{15}H_{23}O(CH_2CH_2O)_7H$ in a mass ratio of 1:0.2:0.5:0.5.

The example was evaluated by using an autoclave, 10 mL of deionized water was prepared and added into the autoclave along with the hydrate promoter in an amount of 1.0% by mass of water in the system, the experimental pressure was 6.5 MPa, and the experimental temperature was 274.7K, it was discovered by the autoclave that the induction time for hydrate was 2.0 min.

Started from the appearance of the hydrate particles in the system, the system pressures were 3952kPa and 3264kPa, respectively when the reaction proceeded to 30 min and 60 min.

It was calculated according to Formula (1) - Formula (3) that the corresponding gas storage capacities were 132.32 V/V and 165.25 V/V, respectively when the reaction proceeded to 30 min and 60 min.

The system temperature was raised to 298.15K after completion of hydrate formation, it was observed through the autoclave that no significant air bubbles appeared in the autoclave during the decomposition process of hydrate; after completion of the decomposition, the actual recovery volume of gas after decomposition was measured by an exhaust valve and a gas flow meter, the gas recovery rate was calculated to be 95% according to Formula (4). In addition, the surface tension (at a temperature of 298.15K) of the liquid phase in the autoclave after completion of the experiment was measured to be 64mN/m, as can be seen, the hydrate promoter in the example had small influence on the surface tension of the water phase, no significant air bubbles appeared during the decomposition process of hydrate, the gas recovery rate was high while ensuring a high gas storage capacity in the hydrate method.

Reference Example 17

The present disclosure provided a hydrate promoter, which was obtained by mixing mono-hydrate cefchlorampin, xanthan gum, Tween 80 and $C_{15}H_{23}O(CH_2CH_2O)_7H$ in a mass ratio of 1:0.2:0.5:0.5.

The example was evaluated by using an autoclave, 10 mL of deionized water was prepared and added into the autoclave along with the hydrate promoter in an amount of 1.0% by mass of water in the system, the experimental pressure was 6.5 MPa, and the experimental temperature was 274.7K, it was discovered by the autoclave that the induction time for

hydrate was 1.6 min.

Started from the appearance of the hydrate particles in the system, the system pressures were 3925kPa and 3108kPa, respectively when the reaction proceeded to 30 min and 60 min.

It was calculated according to Formula (1) - Formula (3) that the corresponding gas storage capacities were 133.59 V/V and 171.35 V/V, respectively when the reaction proceeded to 30 min and 60 min.

The system temperature was raised to 298.15K after completion of hydrate formation, it was observed through the autoclave that no significant air bubbles appeared in the autoclave during the decomposition process of hydrate; after completion of the decomposition, the actual recovery volume of gas after decomposition was measured by an exhaust valve and a gas flow meter, the gas recovery rate was calculated to be 96% according to Formula (4). In addition, the surface tension (at a temperature of 298.15K) of the liquid phase in the autoclave after completion of the experiment was measured to be 65mN/m, as can be seen, the hydrate promoter in the example had small influence on the surface tension of the water phase, no significant air bubbles appeared during the decomposition process of hydrate, the gas recovery rate was high while ensuring a high gas storage capacity in the hydrate method.

Reference Example 18

The present disclosure provided a hydrate promoter, which was obtained by mixing mono-hydrate cefchlorampin, xanthan gum, Tween 80 and $C_{15}H_{23}O(CH_2CH_2O)_7H$ in a mass ratio of 1:0.2:0.5: 0.5.

The example was evaluated by using an autoclave, a (diesel + water) system with a water content of 20 vol% was prepared and added into the autoclave along with the hydrate promoter in an amount of 1.0% by mass of water in the system, the experimental pressure was 6.5 MPa, and the experimental temperature was 274.7K, it was discovered by the autoclave that the induction time for hydrate was 10 min.

Started from the appearance of the hydrate particles in the system, the system pressures were 6084kPa and 5950kPa, respectively when the reaction proceeded to 30 min and 60 min.

It was calculated according to Formula (1) - Formula (3) that the corresponding gas storage capacities were 134.85 V/V and 169.38 V/V, respectively when the reaction proceeded to 30 min and 60 min.

The system temperature was raised to 298.15K after completion of hydrate formation, it was observed through the autoclave that no significant air bubbles appeared in the autoclave during the decomposition process of hydrate; after completion of the decomposition, the actual recovery volume of gas after decomposition was measured by an exhaust valve and a gas flow meter, the gas recovery rate was calculated to be 92% according to Formula (4), no significant air bubbles appeared during the decomposition process of hydrate, the gas recovery rate was high while ensuring a high gas storage capacity in the hydrate method.

Reference Example 19

The present disclosure provided a hydrate promoter, which was obtained by mixing mono-hydrate cefchlorampin, xanthan gum, Tween 80 and $C_{15}H_{23}O(CH_2CH_2O)_7H$ in a mass ratio of 1:0.2:0.5:0.5.

The example was evaluated by using an autoclave, a (diesel + water) system with a water content of 80 vol% was prepared and added into the autoclave along with the hydrate promoter in an amount of 1.0% by mass of water in the system, the experimental pressure was 6.5 MPa, and the experimental temperature was 274.7K, it was discovered by the autoclave that the induction time for hydrate was 8 min.

Started from the appearance of the hydrate particles in the system, the system pressures were 4582kPa and 4038kPa, respectively when the reaction proceeded to 30 min and 60 min.

It was calculated according to Formula (1) - Formula (3) that the corresponding gas storage capacities were 130.42 V/V and 161.33 V/V, respectively when the reaction proceeded to 30 min and 60 min.

The system temperature was raised to 298.15K after completion of hydrate formation, it was observed through the autoclave that no significant air bubbles appeared in the autoclave during the decomposition process of hydrate; after completion of the decomposition, the actual recovery volume of gas after decomposition was measured by an exhaust valve and a gas flow meter, the gas recovery rate was calculated to be 94% according to Formula (4), no significant air bubbles appeared during the decomposition process of hydrate, the gas recovery rate was high while ensuring a high gas storage capacity in the hydrate method.

Reference Example 20

The present disclosure provided a hydrate promoter, which was obtained by mixing mono-hydrate cefchlorampin and $C_{15}H_{23}O(CH_2CH_2O)_7H$ in a mass ratio of 1:1. That is, as compared with Example 1, the example did not contain xanthan gum and Tween 60.

The example was evaluated by using an autoclave, 10 mL of deionized water was prepared and added into the

autoclave along with the hydrate promoter in an amount of 1.0% by mass of water in the system, the experimental pressure was 6.5 MPa, and the experimental temperature was 274.7K, it was discovered by the autoclave that the induction time for hydrate was 3.5 min.

Started from the appearance of the hydrate particles in the system, the system pressures were 4166kPa and 3358kPa, respectively when the reaction proceeded to 30 min and 60 min.

It was calculated according to Formula (1) - Formula (3) that the corresponding gas storage capacities were 122.19 V/V and 160.32 V/V, respectively when the reaction proceeded to 30 min and 60 min.

The system temperature was raised to 298.15K after completion of hydrate formation, it was observed through the autoclave that no significant air bubbles appeared in the autoclave during the decomposition process of hydrate; after completion of the decomposition, the actual recovery volume of gas after decomposition was measured by an exhaust valve and a gas flow meter, the gas recovery rate was calculated to be 93% according to Formula (4). In addition, the surface tension (at a temperature of 298.15K) of the liquid phase in the autoclave after completion of the experiment was measured to be 62mN/m, as can be seen, the hydrate promoter in the example had small influence on the surface tension of the water phase, no significant air bubbles appeared during the decomposition process of hydrate, the gas recovery rate was high while ensuring a high gas storage capacity in the hydrate method.

Reference Example 21

The present disclosure provided a hydrate promoter, which was obtained by mixing mono-hydrate cefchlorampin, xanthan gum, Tween 60 and $C_{15}H_{23}O(CH_2CH_2O)_7H$ in a mass ratio of 1:0.2:0.5:0.5.

The example was evaluated by using an autoclave, 10 mL of deionized water was prepared and added into the autoclave along with the hydrate promoter in an amount of 2.0% by mass of water in the system, the experimental pressure was 6.5 MPa, and the experimental temperature was 274.7K, it was discovered by the autoclave that the induction time for hydrate was 0.85 min.

Started from the appearance of the hydrate particles in the system, the system pressures were 3728kPa and 3050kPa, respectively when the reaction proceeded to 30 min and 60 min.

It was calculated according to Formula (1) - Formula (3) that the corresponding gas storage capacities were 142.81 V/V and 173.58 V/V, respectively when the reaction proceeded to 30 min and 60 min.

The system temperature was raised to 298.15K after completion of hydrate formation, it was observed through the autoclave that no significant air bubbles appeared in the autoclave during the decomposition process of hydrate; after completion of the decomposition, the actual recovery volume of gas after decomposition was measured by an exhaust valve and a gas flow meter, the gas recovery rate was calculated to be 97% according to Formula (4). In addition, the surface tension (at a temperature of 298.15K) of the liquid phase in the autoclave after completion of the experiment was measured to be 65mN/m, as can be seen, the hydrate promoter in the example had small influence on the surface tension of the water phase, no significant air bubbles appeared during the decomposition process of hydrate, the gas recovery rate was high while ensuring a high gas storage capacity in the hydrate method.

Reference Example 22

The present disclosure provided a hydrate promoter, which was obtained by mixing mono-hydrate cefchlorampin, Tween 60 and $C_{15}H_{23}O(CH_2CH_2O)_7H$ in a mass ratio of 1:0.5:0.5. That is, the hydrate promoter did not contain xanthan gum.

The example was evaluated by using an autoclave, 10 mL of deionized water was prepared and added into the autoclave along with the hydrate promoter in an amount of 1.0% by mass of water in the system, the experimental pressure was 6.5 MPa, and the experimental temperature was 274.7K, it was discovered by the autoclave that the induction time for hydrate was 2.6 min.

Started from the appearance of the hydrate particles in the system, the system pressures were 4089kPa and 3342kPa, respectively when the reaction proceeded to 30 min and 60 min.

It was calculated according to Formula (1) - Formula (3) that the corresponding gas storage capacities were 125.85 V/V and 160.68 V/V, respectively when the reaction proceeded to 30 min and 60 min.

The system temperature was raised to 298.15K after completion of hydrate formation, it was observed through the autoclave that no significant air bubbles appeared in the autoclave during the decomposition process of hydrate; after completion of the decomposition, the actual recovery volume of gas after decomposition was measured by an exhaust valve and a gas flow meter, the gas recovery rate was calculated to be 94% according to Formula (4). In addition, the surface tension (at a temperature of 298.15K) of the liquid phase in the autoclave after completion of the experiment was measured to be 63mN/m, as can be seen, the hydrate promoter in the example had small influence on the surface tension of the water phase, no significant air bubbles appeared during the decomposition process of hydrate, the gas recovery rate was high while ensuring a high gas storage capacity in the hydrate method.

Reference Example 23

The present disclosure provided a hydrate promoter, which was obtained by mixing mono-hydrate cefchlorampin, xanthan gum, Tween 60 and $C_{15}H_{23}O(CH_2CH_2O)_7H$ in a mass ratio of 1:11:2.5:2.5.

The example was evaluated by using an autoclave, 10 mL of deionized water was prepared and added into the autoclave along with the hydrate promoter in an amount of 1.0% by mass of water in the system, the experimental pressure was 6.5 MPa, and the experimental temperature was 274.7K, it was discovered by the autoclave that the induction time for hydrate was 1.05 min.

Started from the appearance of the hydrate particles in the system, the system pressures were 3812kPa and 3098kPa, respectively when the reaction proceeded to 30 min and 60 min.

It was calculated according to Formula (1) - Formula (3) that the corresponding gas storage capacities were 138.91 V/V and 171.81 V/V, respectively when the reaction proceeded to 30 min and 60 min.

The system temperature was raised to 298.15K after completion of hydrate formation, it was observed through the autoclave that no significant air bubbles appeared in the autoclave during the decomposition process of hydrate; after completion of the decomposition, the actual recovery volume of gas after decomposition was measured by an exhaust valve and a gas flow meter, the gas recovery rate was calculated to be 96% according to Formula (4). In addition, the surface tension (at a temperature of 298.15K) of the liquid phase in the autoclave after completion of the experiment was measured to be 65mN/m, as can be seen, the hydrate promoter in the example had small influence on the surface tension of the water phase, no significant air bubbles appeared during the decomposition process of hydrate, the gas recovery rate was high while ensuring a high gas storage capacity in the hydrate method.

Reference Example 24

The present disclosure provided a hydrate promoter, which was obtained by mixing mono-hydrate cefchlorampin, xanthan gum, Tween 60 and $C_{15}H_{23}O(CH_2CH_2O)_7H$ in a mass ratio of 1:0.3:1:1.

The example was evaluated by using an autoclave, 10 mL of deionized water was prepared and added into the autoclave along with the hydrate promoter in an amount of 1.0% by mass of water in the system, the experimental pressure was 6.5 MPa, and the experimental temperature was 274.7K, it was discovered by the autoclave that the induction time for hydrate was 0.95 min.

Started from the appearance of the hydrate particles in the system, the system pressures were 3786kPa and 3058kPa, respectively when the reaction proceeded to 30 min and 60 min.

It was calculated according to Formula (1) - Formula (3) that the corresponding gas storage capacities were 140.12 V/V and 173.61 V/V, respectively when the reaction proceeded to 30 min and 60 min.

The system temperature was raised to 298.15K after completion of hydrate formation, it was observed through the autoclave that no significant air bubbles appeared in the autoclave during the decomposition process of hydrate; after completion of the decomposition, the actual recovery volume of gas after decomposition was measured by an exhaust valve and a gas flow meter, the gas recovery rate was calculated to be 95% according to Formula (4). In addition, the surface tension (at a temperature of 298.15K) of the liquid phase in the autoclave after completion of the experiment was measured to be 63mN/m, as can be seen, the hydrate promoter in the example had small influence on the surface tension of the water phase, no significant air bubbles appeared during the decomposition process of hydrate, the gas recovery rate was high while ensuring a high gas storage capacity in the hydrate method.

Reference Example 25

The present disclosure provided a hydrate promoter, which was obtained by mixing mono-hydrate cefchlorampin, xanthan gum, Tween 60 and $C_{15}H_{23}O(CH_2CH_2O)_7H$ in a mass ratio of 1:0.6:2:2.

The example was evaluated by using an autoclave, 10 mL of deionized water was prepared and added into the autoclave along with the hydrate promoter in an amount of 1.0% by mass of water in the system, the experimental pressure was 6.5 MPa, and the experimental temperature was 274.7K, it was discovered by the autoclave that the induction time for hydrate was 0.90 min.

Started from the appearance of the hydrate particles in the system, the system pressures were 3775kPa and 3026kPa, respectively when the reaction proceeded to 30 min and 60 min.

It was calculated according to Formula (1) - Formula (3) that the corresponding gas storage capacities were 140.63 V/V and 174.05 V/V, respectively when the reaction proceeded to 30 min and 60 min.

The system temperature was raised to 298.15K after completion of hydrate formation, it was observed through the autoclave that no significant air bubbles appeared in the autoclave during the decomposition process of hydrate; after completion of the decomposition, the actual recovery volume of gas after decomposition was measured by an exhaust valve and a gas flow meter, the gas recovery rate was calculated to be 96% according to Formula (4). In addition, the

surface tension (at a temperature of 298.15K) of the liquid phase in the autoclave after completion of the experiment was measured to be 65mN/m, as can be seen, the hydrate promoter in the example had small influence on the surface tension of the water phase, no significant air bubbles appeared during the decomposition process of hydrate, the gas recovery rate was high while ensuring a high gas storage capacity in the hydrate method.

Reference Example 26

The present disclosure provided a hydrate promoter, which was obtained by mixing mono-hydrate cefchlorampin, xanthan gum, Tween 60 and $C_{15}H_{23}O(CH_2CH_2O)_7H$ in a mass ratio of 1:0.2:2.5:0.5.

The example was evaluated by using an autoclave, 10 mL of deionized water was prepared and added into the autoclave along with the hydrate promoter in an amount of 1.0% by mass of water in the system, the experimental pressure was 6.5 MPa, and the experimental temperature was 274.7K, it was discovered by the autoclave that the induction time for hydrate was 1.12 min.

Started from the appearance of the hydrate particles in the system, the system pressures were 3850kPa and 3152kPa, respectively when the reaction proceeded to 30 min and 60 min.

It was calculated according to Formula (1) - Formula (3) that the corresponding gas storage capacities were 137.12 V/V and 169.35 V/V, respectively when the reaction proceeded to 30 min and 60 min.

The system temperature was raised to 298.15K after completion of hydrate formation, it was observed through the autoclave that no significant air bubbles appeared in the autoclave during the decomposition process of hydrate; after completion of the decomposition, the actual recovery volume of gas after decomposition was measured by an exhaust valve and a gas flow meter, the gas recovery rate was calculated to be 95% according to Formula (4). In addition, the surface tension (at a temperature of 298.15K) of the liquid phase in the autoclave after completion of the experiment was measured to be 64mN/m, as can be seen, the hydrate promoter in the example had small influence on the surface tension of the water phase, no significant air bubbles appeared during the decomposition process of hydrate, the gas recovery rate was high while ensuring a high gas storage capacity in the hydrate method.

Reference Example 27

The example was evaluated by using the hydrate promoter autoclave of Example 26, a (diesel + water) system with a water content of 80 vol% was prepared and added into the autoclave along with the hydrate promoter in an amount of 1.0% by mass of water in the system, the experimental pressure was 6.5 MPa, and the experimental temperature was 274.7K, it was discovered that the induction time for hydrate was 7.5 min.

Started from the appearance of the hydrate particles in the system, the system pressures were 4445kPa and 4080kPa, respectively when the reaction proceeded to 30 min and 60 min.

It was calculated according to Formula (1) - Formula (3) that the corresponding gas storage capacities were 132.98 V/V and 161.02 V/V, respectively when the reaction proceeded to 30 min and 60 min.

The system temperature was raised to 298.15K after completion of hydrate formation, it was observed through the autoclave that no significant air bubbles appeared in the autoclave during the decomposition process of hydrate; after completion of the decomposition, the actual recovery volume of gas after decomposition was measured by an exhaust valve and a gas flow meter, the gas recovery rate was calculated to be 64% according to Formula (4), no significant air bubbles appeared during the decomposition process of hydrate, the gas recovery rate was high while ensuring a high gas storage capacity in the hydrate method.

Reference Example 28

The present disclosure provided a hydrate promoter, which was obtained by mixing mono-hydrate cefchlorampin, dodecyl glycoside, Tween 60 and Span 20 in a mass ratio of 1:0.2:0.5:0.5.

The example was evaluated by using an autoclave, 10 mL of deionized water was prepared and added into the autoclave along with the hydrate promoter in an amount of 1.0% by mass of water in the system, the experimental pressure was 6.5 MPa, and the experimental temperature was 274.7K, it was discovered by the autoclave that the induction time for hydrate was 3 min.

Started from the appearance of the hydrate particles in the system, the system pressures were 4120kPa and 3354kPa, respectively when the reaction proceeded to 30 min and 60 min.

It was calculated according to Formula (1) - Formula (3) that the corresponding gas storage capacities were 124.38 V/V and 160.13 V/V, respectively when the reaction proceeded to 30 min and 60 min.

The system temperature was raised to 298.15K after completion of hydrate formation, it was observed through the autoclave that no significant air bubbles appeared in the autoclave during the decomposition process of hydrate; after completion of the decomposition, the actual recovery volume of gas after decomposition was measured by an exhaust

valve and a gas flow meter, the gas recovery rate was calculated to be 95% according to Formula (4). In addition, the surface tension (at a temperature of 298.15K) of the liquid phase in the autoclave after completion of the experiment was measured to be 63mN/m, as can be seen, the hydrate promoter in the example had small influence on the surface tension of the water phase, no significant air bubbles appeared during the decomposition process of hydrate, the gas recovery rate was high while ensuring a high gas storage capacity in the hydrate method.

Example 29

The hydrate promoter was provided according to the method of Example 14, except that a mass ratio of monohydrate cefchlorampin, xanthan gum, Tween 60 and $C_{15}H_{23}O(CH_2CH_2O)_7H$ was1:0.1:0.05:0.05.

Validation was carried out according to the method of Example 14, the induction time of hydrate was discovered to be 2.4min, the corresponding gas storage capacities were 129.85 V/V and 161.25 V/V, respectively when the reaction proceeded to 30 min and 60 min.

The gas recovery rate was 95%, and the surface tension (at a temperature of 298.15K) of the liquid phase in the autoclave after completion of the experiment was 64mN/m.

Reference Example 30

The hydrate promoter was provided according to the method of Example 14, except that a mass ratio of monohydrate cefchlorampin, xanthan gum, Tween 60 and $C_{15}H_{23}O(CH_2CH_2O)_7H$ was1:5:5:5.

Validation was carried out according to the method of Example 14, the induction time of hydrate was discovered to be 2.5min, the corresponding gas storage capacities were 128.72 V/V and 161.83 V/V, respectively when the reaction proceeded to 30 min and 60 min.

The gas recovery rate was 94%, and the surface tension (at a temperature of 298.15K) of the liquid phase in the autoclave after completion of the experiment was 64mN/m.

Example 31

The hydrate promoter was configured according to the method of Example 1, except that the hydrate promoter further comprised an anti-agglomerant; the hydrate promoter was obtained by mixing penicillin sodium, dodecyl dimethyl betaine, $C_{15}H_{25}O(CH_2CH_2O)_5H$, $Na_2CO_3$ and atorvastatin, wherein the mass ratio of penicillin sodium, dodecyl dimethyl betaine, $C_{15}H_{25}O(CH_2CH_2O)_5H$ and $Na_2CO_3$ was 1:0.05:0.05:0.5; the added mass of atorvastatin was 5 times of the total mass of penicillin sodium, dodecyl dimethyl betaine, $C_{15}H_{25}O(CH_2CH_2O)_5H$ and $Na_2CO_3$.

The example was evaluated by using an autoclave, 10 mL of deionized water was prepared and added into the autoclave along with the hydrate promoter in an amount that the total mass of penicillin sodium, dodecyl dimethyl betaine, $C_{15}H_{25}O(CH_2CH_2O)_5H$ and $Na_2CO_3$ was 0.5% by mass of water in the system, the experimental pressure was 7.0 MPa, and the experimental temperature was 276.2K, it was discovered by the autoclave that the induction time for hydrate was 1.15 min.

The corresponding gas storage capacity was 169.25 V/V when the reaction proceeded to 60 min.

The system temperature was raised to 298.15K after completion of hydrate formation, it was observed through the autoclave that no significant air bubbles appeared in the autoclave during the decomposition process of hydrate; after completion of the decomposition, the actual recovery volume of gas after decomposition was measured by an exhaust valve and a gas flow meter, the gas recovery rate was calculated to be 98% according to Formula (4). In addition, the surface tension (at a temperature of 298.15K) of the liquid phase in the autoclave after completion of the experiment was measured to be 68mN/m, as can be seen, the hydrate promoter in the example had small influence on the surface tension of the water phase, no significant air bubbles appeared during the decomposition process of hydrate, the gas recovery rate was high while ensuring a high gas storage capacity in the hydrate method.

Example 32

The hydrate promoter was configured according to the method of Example 1, except that the hydrate promoter further comprised an effervescent agent; that is, the hydrate promoter comprised a first part and a second part, the first part of the hydrate promoter was obtained by mixing penicillin sodium, dodecyl dimethyl betaine, $C_{15}H_{25}O(CH_2CH_2O)_5H$ and $Na_2CO_3$ in a amass ratio of 1:0.05:0.05:0.5; the second part of the hydrate promoter was an effervescent agent, the used amount of the effervescent agent by mass is 0.025 times of the used amount of mass of the first part of the hydrate promoter (citric acid + sodium bicarbonate in a mass ratio of 1: 1).

The example was evaluated by using an autoclave, 10 mL of deionized water was prepared and added into the autoclave along with the first part of the hydrate promoter in an amount of 0.5% by mass of water in the system, the

experimental pressure was 7.0 MPa, and the experimental temperature was 276.2K, the effervescent agent was added when the system conditions satisfied the experimental conditions, it was discovered by the autoclave that the induction time for hydrate was 1.2 min.

The corresponding gas storage capacity was 168.55 V/V when the reaction proceeded to 60min.

The system temperature was raised to 298.15K after completion of hydrate formation, it was observed through the autoclave that no significant air bubbles appeared in the autoclave during the decomposition process of hydrate; after completion of the decomposition, the actual recovery volume of gas after decomposition was measured by an exhaust valve and a gas flow meter, the gas recovery rate was calculated to be 98% according to Formula (4). In addition, the surface tension (at a temperature of 298.15K) of the liquid phase in the autoclave after completion of the experiment was measured to be 69mN/m, as can be seen, the hydrate promoter in the example had small influence on the surface tension of the water phase, no significant air bubbles appeared during the decomposition process of hydrate, the gas recovery rate was high while ensuring a high gas storage capacity in the hydrate method.

Example 33

The hydrate promoter was configured according to the method of Example 1, except that the penicillin sodium in Example 1 was replaced with the ampicillin sodium.

Validation was carried out according to the method of Example 1, the induction time of hydrate was discovered to be 0.85min, the corresponding gas storage capacities were 137.54 V/V, 168.52 V/V and 172.15 V/V, respectively when the reaction proceeded to 30 min, 60 min and 120 min.

The gas recovery rate was 99%, and the surface tension (at a temperature of 298.15K) of the liquid phase after completion of the experiment was 69mN/m.

Reference Example 34

The hydrate promoter was configured according to the method of Example 14, except that the mono-hydrate cefchlorampin in Example 14 was replaced with the cefadroxil.

Validation was carried out according to the method of Example 1, the induction time of hydrate was discovered to be 1.12min, the corresponding gas storage capacities were 136.35 V/V and 169.85 V/V, respectively when the reaction proceeded to 30 min and 60 min.

The gas recovery rate was 97%, and the surface tension (at a temperature of 298.15K) of the liquid phase after completion of the experiment was 65mN/m.

Comparative Example 1

10mL of deionized water was added into an autoclave without adding any hydrate promoter, the experimental pressure was 7.0MPa, the experimental temperature was maintained at 276.2K, it was discovered by the autoclave that the induction time for hydrate was 6.5 min; started from the appearance of the hydrate particles in the system, the system pressures were 6510.26kPa, 6431.68kPa and 6388.75kPa, respectively when the reaction proceeded to 30 min, 60min and 120 min; it was calculated according to Formula (1) - Formula (3) that the corresponding gas storage capacities were 29.29 V/V, 33.43 V/V and 32.85 V/V, respectively when the reaction proceeded to 30 min, 60min and 120 min.

The system temperature was raised to 298.15K after completion of hydrate formation, it was observed through the autoclave that no significant air bubbles appeared in the autoclave during the decomposition process of hydrate; after completion of the decomposition, the actual recovery volume of gas after decomposition was measured by an exhaust valve and a gas flow meter, the gas recovery rate was calculated to be 96% according to Formula (4). In addition, the surface tension (at a temperature of 298.15K) of the liquid phase in the autoclave after completion of the experiment was measured to be 71mN/m, as can be seen, the gas storage capacity of the hydrate in the Comparative Example was too small.

Comparative Example 2

The hydrate promoter was prepared by mixing cyclopentane and 2-butyloctylsulfate sodium (GC12S) in a mass ratio of 1:0.1; the hydrate promoter was used as a comparative hydrate promoter, it was formulated into 10mL of aqueous solution with a concentration of 0.5% and then added into an autoclave, the experimental pressure was 7.0MPa, the experimental temperature was maintained at 276.2K, it was discovered by the autoclave that the induction time for hydrate was 5.25 min; started from the appearance of the hydrate particles in the system, the system pressures were 5530.52kPa, 5038.36kPa and 4782.15kPa, respectively when the reaction proceeded to 30 min, 60min and 120 min; it was calculated according to Formula (1) - Formula (3) that the corresponding gas storage capacities were 67.78 V/V, 91.84 V/V and

104.18 V/V, respectively when the reaction proceeded to 30 min, 60min and 120 min.

The system temperature was raised to 298.15K after completion of hydrate formation, it was observed through the autoclave that a small amount of air bubbles appeared in the autoclave during the decomposition process of hydrate; after completion of the decomposition, the actual recovery volume of gas after decomposition was measured by an exhaust valve and a gas flow meter, the gas recovery rate was calculated to be 11% according to Formula (4). In addition, the surface tension (at a temperature of 298.15K) of the liquid phase in the autoclave after completion of the experiment was measured to be 38mN/m, as can be seen, although the gas storage capacity of the Comparative Example 2 was slightly improved compared to the Comparative Example 1, the overall generation rate of the hydrate formation process was still slow, and a large amount of air bubbles still appeared during the decomposition process of hydrate, the gas recovery rate was low and was not suitable for the large-scale application.

Comparative Example 3

The conventional anionic surfactant dodecyl sulfate sodium was used as a comparative hydrate promoter, it was formulated into 10mL of aqueous solution with a concentration of 0.05% and then added into an autoclave, the experimental pressure was 7.0MPa, the experimental temperature was maintained at 276.2K, it was discovered by the autoclave that the induction time for hydrate was 3.75 min; started from the appearance of the hydrate particles in the system, the system pressures were 4486.15kPa, 4126.42kPa and 3813.61kPa, respectively when the reaction proceeded to 30 min, 60min and 120 min; it was calculated according to Formula (1) - Formula (3) that the corresponding gas storage capacities were 131.63 V/V, 148.87 V/V and 163.04 V/V, respectively when the reaction proceeded to 30 min, 60min and 120 min.

The system temperature was raised to 298.15K after completion of hydrate formation, it was observed through the autoclave that a large amount of air bubbles appeared in the autoclave during the decomposition process of hydrate and the whole autoclave was filled with air bubbles; after completion of the decomposition, the gas cannot be effectively recovered by an exhaust valve and a gas flow meter, the gas recovery rate was calculated to be 5% according to Formula (4). In addition, the surface tension (at a temperature of 298.15K) of the liquid phase in the autoclave after completion of the experiment was measured to be 28mN/m, as can be seen, the dodecylsulfate sodium in this Comparative Example was a typical anionic surfactant, the gas release during the decomposition process of hydrate causes the formation of a large amount of gas bubbles, so that the decomposed gas cannot be effectively recovered and reused, the hydrate promoter was not suitable for the large-scale application.

In summary, it can be derived from the data of Examples 1-13 and Comparative Examples 1-3, the induction time of hydrate in Examples 1-5 was within a range of 1.25-2.5min, the induction time of hydrate in Comparative Example 1 was 6.5min, the induction time of hydrate in Comparative Example 2 was 5.25 min, the induction time of hydrate was 2.75 min when the conventional anionic surfactant was used as the hydrate promoter in Comparative Example 3; the hydrate promoter provided by the present disclosure can effectively shorten the induction time of hydrate, and formation pressure of hydrate was small, the atmospheric storage and transportation of the hydrate was feasible, such that the storage and transportation process was in accordance with the safety standards. Moreover, the gas recovery rates of the present disclosure were 90% or more, air bubbles did not appear during the decomposition process, thus the decomposition efficiency of hydrate can be improved.

In addition, in order to facilitate the detailed comparison of the changes in the system pressure and gas storage capacity during the generation process of a hydrate in various Comparative Examples and Examples, FIG. 1 and FIG. 2 illustrated the specific conditions of respective stage in Example 1 and Comparative Examples 1-3, FIG. 3 illustrated the comparison conditions of generating air bubbles in the autoclave after the complete decomposition of hydrate in Comparative Examples 1-3 and Example 1.

Comparative Example 4

10mL of deionized water without adding any hydrate promoter was added into an autoclave, the experimental pressure was 6.5MPa, the experimental temperature was maintained at 274.7K, it was discovered by the autoclave that the induction time for hydrate was 18 min.

Started from the appearance of the hydrate particles in the system, the system pressures were 6388kPa and 6235kPa, respectively when the reaction proceeded to 30 min and 60min.

It was calculated according to Formula (1) - Formula (3) that the corresponding gas storage capacities were 11.15 V/V and 20.36 V/V, respectively when the reaction proceeded to 30 min and 60min.

The system temperature was raised to 298.15K after completion of hydrate formation, it was observed through the autoclave that no significant air bubbles appeared in the autoclave during the decomposition process of hydrate; after completion of the decomposition, the actual recovery volume of gas after decomposition was measured by an exhaust valve and a gas flow meter, the gas recovery rate was calculated to be 94% according to Formula (4). In addition, the

surface tension (at a temperature of 298.15K) of the liquid phase in the autoclave after completion of the experiment was measured to be 71mN/m.

Comparative Example 5

10mL of a (diesel + water) system having a water content of 20 vol% without adding any hydrate promoter was accurately measured and taken, and then added into an autoclave, the experimental pressure was 6.5 MPa, and the experimental temperature was 274.7K, it was discovered by the autoclave that the induction time for hydrate was 32 min.
Started from the appearance of the hydrate particles in the system, the system pressures were 6050kPa and 5975kPa, respectively when the reaction proceeded to 30 min and 60 min.
It was calculated according to Formula (1) - Formula (3) that the corresponding gas storage capacities were 35.83 V/V and 40.45 V/V, respectively when the reaction proceeded to 30 min and 60 min.
The system temperature was raised to 298.15K after completion of hydrate formation, it was observed through the autoclave that no significant air bubbles appeared in the autoclave during the decomposition process of hydrate; after completion of the decomposition, the actual recovery volume of gas after decomposition was measured by an exhaust valve and a gas flow meter, the gas recovery rate was calculated to be 82% according to Formula (4).

Comparative Example 6

10mL of a (diesel + water) system having a water content of 80 vol% without adding any hydrate promoter was accurately measured and taken, and then added into an autoclave, the experimental pressure was 6.5 MPa, and the experimental temperature was 274.7K, it was discovered by the autoclave that the induction time for hydrate was 25 min.
Started from the appearance of the hydrate particles in the system, the system pressures were 6025kPa and 5940kPa, respectively when the reaction proceeded to 30 min and 60 min.
It was calculated according to Formula (1) - Formula (3) that the corresponding gas storage capacities were 27.74 V/V and 34.40 V/V, respectively when the reaction proceeded to 30 min and 60 min.
The system temperature was raised to 298.15K after completion of hydrate formation, it was observed through the autoclave that no significant air bubbles appeared in the autoclave during the decomposition process of hydrate; after completion of the decomposition, the actual recovery volume of gas after decomposition was measured by an exhaust valve and a gas flow meter, the gas recovery rate was calculated to be 80% according to Formula (4).

Comparative Example 7

A hydrate promoter was obtained by mixing chloramphenicol, xanthan gum, Tween 60 and $C_{15}H_{23}O(CH_2CH_2O)_7H$ in a mass ratio of 1:0.2:0.5:0.5.
The example was evaluated by using an autoclave, 10 mL of deionized water was prepared and added into the autoclave along with the hydrate promoter in an amount of 1.0% by mass of water in the system, the experimental pressure was 6.5 MPa, and the experimental temperature was 274.7K, it was discovered by the autoclave that the induction time for hydrate was 17.5 min.
Started from the appearance of the hydrate particles in the system, the system pressures were 4325kPa and 3652kPa, respectively when the reaction proceeded to 30 min and 60 min.
It was calculated according to Formula (1) - Formula (3) that the corresponding gas storage capacities were 102.87 V/V and 136.58 V/V, respectively when the reaction proceeded to 30 min and 60 min.
The system temperature was raised to 298.15K after completion of hydrate formation, it was observed through the autoclave that a large number of air bubbles appeared in the autoclave during the decomposition process of hydrate, and the whole autoclave was filled with the air bubbles; after completion of the decomposition, the gas cannot be effectively recovered through an exhaust valve and a gas flow meter, the gas recovery rate was calculated to be 3% according to Formula (4). In addition, the surface tension (at a temperature of 298.15K) of the liquid phase in the autoclave after completion of the experiment was measured to be 29mN/m.
It is demonstrated from the verification of technical effects when the hydrate promoter of the present disclosure is used in the aqueous phase and gas phase coexistence system, the induction time is greatly shortened, the system pressure is significantly reduced when the reaction proceeds to 30 min and 60 min, the induction time of Example 24 may be as low as 0.95 min, the system pressures are 3786kPa and 3058kPa, respectively when the reaction proceeds to 30 min and 60 min. It demonstrates that the hydrate promoter of the present disclosure in the aqueous phase and gas phase coexistence system can effectively enhance the generation process of hydrate, significantly reduce the nucleation time of gas hydrate, and improve the hydrate growth rate, thus the hydrate promoter has the advantages of desirable promotion effect and high gas storage capacity.
When the hydrate promoter of the present disclosure is used in an oil phase - gas phase - water phase

coexistence system, the induction time is greatly shortened, the system pressure is significantly reduced when the reaction proceeds to 30 min and 60 min, the induction time of Example 27 may be as low as 7.5 min, the system pressures are 4445kPa and 4080kPa, respectively when the reaction proceeds to 30 min and 60 min. It demonstrates that the hydrate promoter of the present disclosure in the oil phase - gas phase - water phase coexistence system can effectively strengthen the generation process of hydrate, significantly reduce the nucleation time of gas hydrate, and improve the hydrate growth rate, thus the hydrate promoter has the advantages of desirable promotion effect and high gas storage capacity.

## Claims

1. A hydrate promoter, comprising a component A which is a substance having a group represented by the Formula I below and a surfactant, wherein a molar ratio of the component A to the surfactant is 1: (0.03-30);

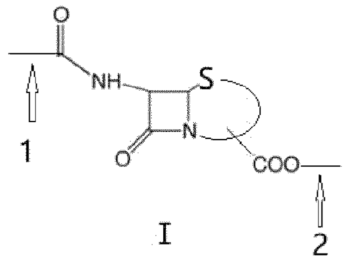

I ,

wherein the reference signs "-" marked by 1, 2 in Formula I are chemical bonds; the cyclic structure having N and S in Formula I is a 4-8 member ring, the group -COO- is located at the ortho-position or meta-position relative to the N atom of the ring in the cyclic structure having N and S; the remaining backbone atoms of the cyclic structure having N and S in Formula I are C atoms;
wherein, the component A is selected from the penicillin compounds, and the surfactant is consisting of a betaine type zwitterionic surfactant and a fatty alcohol polyoxyethylene ether non-ionic surfactant;
wherein a mass ratio of the betaine type zwitterionic surfactant to the fatty alcohol polyoxyethylene ether non-ionic surfactant is (0.1-10):1.

2. The hydrate promoter of claim 1, wherein the mass ratio of the component A and the surfactant is 1: (0.1-10); preferably 1: (0.1-5).

3. The hydrate promoter of claim 1 or 2, wherein the cyclic structure having N and S in Formula I is a saturated 4-8 member ring or a 4-8 member ring having 1-3 C-C unsaturated bonds.

4. The hydrate promoter of any one of claims 1-3, wherein the cyclic structure having N and S in Formula I is a 5-6 member ring, and the remaining backbone atoms of the cyclic structure having N and S are C atoms;

preferably, the group -COO- is located at ortho-position relative to the N atom of the ring in the cyclic structure having N and S;
further preferably, the component A is selected from penicillin compounds having a group represented by

Formula III;

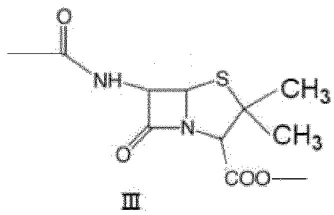

III .

5. The hydrate promoter of any one of claims 1-4, wherein the component A is selected from a penicillin antibiotic and/or a

salt corresponding to the penicillin antibiotic;

preferably, the salt corresponding to the penicillin antibiotic is at least one selected from the group consisting of sodium salt, potassium salt and ammonium salt;
further preferably, the penicillin antibiotic is at least one selected from the group consisting of penicillin G class, penicillin V class, enzyme resistant penicillin, ampicillin class and pseudomonas resistant penicillin;
still further preferably, the penicillin compound is at least one selected from the group consisting of ampicillin sodium, ampicillin, penicillin potassium, carbenicillin sodium, penicillin sodium and oxacillin sodium.

**6.** The hydrate promoter of any one of claims 1-5, wherein the hydrate promoter further comprises an adjuvant

selected from a polysaccharide gum and/or a water-soluble inorganic salt;
preferably, a mass ratio of the component A to the adjuvant is 1: (0.1-5);
preferably, the component A is selected from the penicillin compounds, the adjuvant is selected from the water-soluble inorganic salts;
preferably, a mass ratio of the penicillin compound, the surfactant and the water-soluble inorganic salt is 1: (0.1-5): (0.1-5), preferably 1: (0.1-1): (0.1-5);
further preferably, the water-soluble inorganic salt is at least one selected from the group consisting of $Na_2CO_3$, $K_2CO_3$, NaCl, KCl and $MgCl_2$.

**7.** The hydrate promoter of any one of claims 1-6, wherein the hydrate promoter further comprises an anti-agglomerant, wherein a molecular structure of the anti-agglomerant contains a carbonyl and/or a hydroxyl and a plurality of cyclic structures;

preferably, the anti-agglomerant contains 3-4 multi-member rings;
further preferably, the multi-member ring is a five-member ring or a six-member ring;
still further preferably, the multi-member ring is selected from phenyl or a group containing at least one of a C-C unsaturated bond and an ester group;
still further preferably, the anti-agglomerant is selected from the statin organics, preferably at least one selected from the group consisting of pravastatin, lovastatin, simvastatin and atorvastatin.

**8.** The hydrate promoter of claim 7, wherein the anti-agglomerant is contained in an amount of 1-10 parts by mass, preferably 3-6 parts by mass, relative to a total amount of 1 part by mass of the component A and the surfactant and optionally the adjuvant contained in the hydrate promoter.

**9.** The hydrate promoter of any one of claims 1-8, wherein the hydrate promoter further comprises an effervescent agent, which is capable of voluntarily releasing gas in water;

preferably, the effervescent agent is used in an amount of 0.01-0.5 parts by mass, preferably 0.02-0.3 parts by mass, relative to a total amount of 1 part by mass of the component A and the surfactant and optionally the adjuvant contained in the hydrate promoter;
further preferably, the effervescent agent comprises an acid source and an alkali source; preferably a mass ratio of the acid source and the alkali source is 1: (0.5-2);
still further preferably, the acid source is at least one selected from the group consisting of citric acid, malic acid, boric acid, tartaric acid and fumaric acid, and the alkali source is selected from a basic carbonate and/or a basic bicarbonate, preferably at least one selected from the group consisting of sodium bicarbonate, sodium carbonate, potassium carbonate and potassium bicarbonate.

**10.** A method for preparing a hydrate, comprising: contacting a gas in an aqueous system with the hydrate promoter of any one of claims 1-9 under hydrate formation conditions.

**11.** The method of claim 10, wherein the hydrate formation conditions comprise: a temperature of -20°C to 50°C; a pressure of 0.1-20MPa.

**12.** The method of claim 10 or 11, wherein a mass ratio of the total mass of the component A and the surfactant and optionally added adjuvant relative to water is (0.1-10):100.

**13.** A hydrate comprising the hydrate promoter of any one of claims 1-9.

## Patentansprüche

1. Hydratpromotor, umfassend eine Komponente A, die eine Substanz ist, die eine durch die nachstehende Formel I dargestellte Gruppe aufweist, und ein Tensid, wobei ein Stoffmengenverhältnis der Komponente A zu dem Tensid 1: (0,03-30) beträgt;

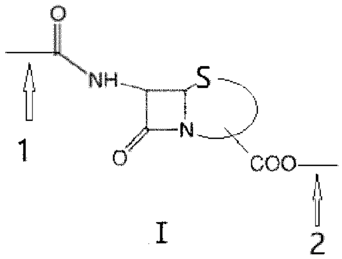

I ,

wobei die in Formel I mit 1, 2 gekennzeichneten Bezugszeichen "-" chemische Bindungen sind; die N und S aufweisende zyklische Struktur in der Formel I ein 4-8-gliedriger Ring ist, die Gruppe -COO- an der ortho-Position oder meta-Position relativ zum N-Atom des Rings in der N und S aufweisenden zyklischen Struktur angeordnet ist; die verbleibenden Gerüstatome der N und S aufweisenden zyklischen Struktur in der Formel I C-Atome sind;
wobei die Komponente A aus den Penicillinverbindungen ausgewählt ist und das Tensid aus einem Betain-Tensid vom Zwitterionen-Typ und einem nichtionischen Fettalkohol-Polyoxyethylenether-Tensid besteht;
wobei ein Massenverhältnis des Betain-Tensids vom Zwitterionen-Typ zu dem nichtionischen Fettalkohol-Polyoxyethylenether-Tensid (0,1-10):1 beträgt.

2. Hydratpromotor nach Anspruch 1, wobei das Massenverhältnis der Komponente A zu dem Tensid 1: (0,1-10); bevorzugt 1: (0,1-5) beträgt.

3. Hydratpromotor nach Anspruch 1 oder 2, wobei die N und S aufweisende zyklische Struktur in der Formel I ein gesättigter 4-8-gliedriger Ring oder ein 4-8-gliedriger Ring mit 1-3 ungesättigten C-C-Bindungen ist.

4. Hydratpromotor nach einem der Ansprüche 1-3, wobei die N und S aufweisende zyklische Struktur in der Formel I ein 5-6-gliedriger Ring ist und die verbleibenden Gerüstatome der N und S aufweisenden zyklischen Struktur C-Atome sind;

bevorzugt, wobei die Gruppe -COO- an der ortho-Position relativ zum N-Atom des Rings in der N und S aufweisenden zyklischen Struktur angeordnet ist;
bevorzugter, wobei die Komponente A aus Penicillinverbindungen ausgewählt ist, die eine durch die Formel III dargestellte Gruppe aufweisen;

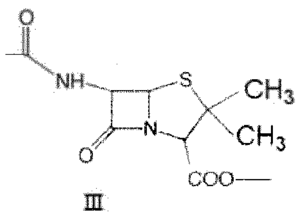

III .

5. Hydratpromotor nach einem der Ansprüche 1-4, wobei die Komponente A aus einem Penicillinantibiotikum und/oder einem dem Penicillinantibiotikum entsprechenden Salz ausgewählt ist;

bevorzugt, wobei das dem Penicillinantibiotikum entsprechende Salz mindestens eines ist, ausgewählt aus der Gruppe bestehend aus Natriumsalz, Kaliumsalz und Ammoniumsalz;
bevorzugter, wobei das Penicillinantibiotikum mindestens eines ist, ausgewählt aus der Gruppe bestehend aus Penicillin-G-Klasse, Penicillin-V-Klasse, enzymresistentem Penicillin, Ampicillin-Klasse und Pseudomonas-resistentem Penicillin;

noch bevorzugter, wobei die Penicillinverbindung mindestens eine ist, ausgewählt aus der Gruppe bestehend aus Ampicillin-Natrium, Ampicillin, Penicillin-Kalium, Carbenicillin-Natrium, Penicillin-Natrium und Oxacillin-Natrium.

**6.** Hydratpromotor nach einem der Ansprüche 1-5, wobei der Hydratpromotor weiter einen Hilfsstoff umfasst, ausgewählt aus einem Polysaccharid-Gummi und/oder einem wasserlöslichen anorganischen Salz;

bevorzugt, wobei ein Massenverhältnis der Komponente A zu dem Hilfsstoff 1: (0,1-5) beträgt;
bevorzugt, wobei die Komponente A aus den Penicillinverbindungen ausgewählt ist, der Hilfsstoff aus den wasserlöslichen anorganischen Salzen ausgewählt ist;
bevorzugt, wobei ein Massenverhältnis der Penicillinverbindung, des Tensids und des wasserlöslichen anorganischen Salzes 1: (0,1-5): (0,1-5), bevorzugt 1: (0,1-1): (0,1-5) beträgt;
bevorzugter, wobei das wasserlösliche anorganische Salz mindestens eines ist, ausgewählt aus der Gruppe bestehend aus $Na_2CO_3$, $K_2CO_3$, NaCl, KCl und $MgCl_2$.

**7.** Hydratpromotor nach einem der Ansprüche 1-6, wobei der Hydratpromotor weiter ein Antiagglomerant umfasst, wobei eine Molekularstruktur des Antiagglomerants eine Carbonylgruppe und/oder eine Hydroxylgruppe und eine Vielzahl zyklischer Strukturen enthält;

bevorzugt, wobei das Antiagglomerant 3-4 mehrgliedrige Ringe enthält;
bevorzugter, wobei der mehrgliedrige Ring ein fünfgliedriger Ring oder ein sechsgliedriger Ring ist;
noch bevorzugter, wobei der mehrgliedrige Ring aus Phenyl oder einer Gruppe ausgewählt ist, die mindestens eine ungesättigte C-C-Bindung und/oder eine Estergruppe enthält;
noch bevorzugter, wobei das Antiagglomerant aus der Gruppe der Statine ausgewählt ist, bevorzugt mindestens eines, ausgewählt aus der Gruppe bestehend aus Pravastatin, Lovastatin, Simvastatin und Atorvastatin, ist.

**8.** Hydratpromotor nach Anspruch 7, wobei das Antiagglomerant in einer Menge von 1-10 Gewichtsteilen, bevorzugt 3-6 Gewichtsteilen, enthalten ist, bezogen auf eine Gesamtmenge von 1 Gewichtsteil der Komponente A und des Tensids und wahlweise des im Hydratpromotor enthaltenen Hilfsstoffs.

**9.** Hydratpromotor nach einem der Ansprüche 1-8, wobei der Hydratpromotor weiter ein Brausemittel umfasst, das in der Lage ist, spontan Gas in Wasser freizusetzen;

bevorzugt, wobei das Brausemittel in einer Menge von 0,01-0,5 Gewichtsteilen, bevorzugt 0,02-0,3 Gewichtsteilen verwendet wird, bezogen auf eine Gesamtmenge von 1 Gewichtsteil der Komponente A und des Tensids und wahlweise des im Hydratpromotor enthaltenen Hilfsstoffs;
bevorzugter, wobei das Brausemittel eine Säurequelle und eine basische Quelle umfasst; bevorzugt, wobei ein Massenverhältnis der Säurequelle zu der basischen Quelle 1: (0.5-2) beträgt;
noch bevorzugter, wobei die Säurequelle mindestens eine ist, ausgewählt aus der Gruppe bestehend aus Zitronensäure, Apfelsäure, Borsäure, Weinsäure und Fumarsäure, und die basische Quelle aus einem basischen Carbonat und/oder einem basischen Hydrogencarbonat ausgewählt ist, bevorzugt mindestens eines, ausgewählt aus der Gruppe bestehend aus Natriumhydrogencarbonat, Natriumcarbonat, Kaliumcarbonat und Kaliumhydrogencarbonat, ist.

**10.** Verfahren zur Herstellung eines Hydrats, umfassend: Inkontaktbringen eines Gases in einem wässrigen System mit dem Hydratpromotor nach einem der Ansprüche 1-9 unter den Bedingungen der Hydratbildung.

**11.** Verfahren nach Anspruch 10, wobei die Bedingungen der Hydratbildung Folgendes umfassen: eine Temperatur von -20 °C bis 50 °C; einen Druck von 0,1-20 MPa.

**12.** Verfahren nach Anspruch 10 oder 11, wobei ein Massenverhältnis der Gesamtmasse der Komponente A und des Tensids und wahlweise zugesetzten Hilfsstoffs zu Wasser (0,1-10):100 beträgt.

**13.** Hydrat, umfassend den Hydratpromotor nach einem der Ansprüche 1-9.

## Revendications

1. Promoteur d'hydrate, comprenant un composant A qui est une substance présentant un groupe représenté par la Formule I ci-dessous et un tensioactif, dans lequel un rapport molaire du composant A au tensioactif est de 1 : (0,03-30) ;

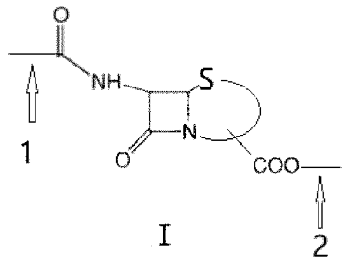

I ,

dans lequel les signes de référence « - » marqués par 1, 2 dans la Formule I sont des liaisons chimiques ; la structure cyclique présentant N et S dans la Formule I est un cycle à 4-8 chaînons, le groupe -COO- est situé en position ortho ou en position méta par rapport à l'atome N du cycle dans la structure cyclique présentant N et S ; les atomes du squelette restants de la structure cyclique présentant N et S dans la Formule I sont des atomes de C ; dans lequel, le composant A est sélectionné parmi les composés de pénicilline, et le tensioactif consiste en un tensioactif zwitterionique de type bétaïne et un tensioactif non ionique d'éther polyoxyéthylénique d'alcool gras ; dans lequel un rapport massique du tensioactif zwitterionique de type bétaïne au tensioactif non ionique d'éther polyoxyéthylénique d'alcool gras est de (0,1-10):1.

2. Promoteur d'hydrate selon la revendication 1, dans lequel le rapport massique du composant A et du tensioactif est de 1 : (0,1-10) ; préférablement 1 : (0,1-5).

3. Promoteur d'hydrate selon la revendication 1 ou la revendication 2, dans lequel la structure cyclique présentant N et S dans la Formule I est un cycle à 4-8 chaînons saturé ou un cycle à 4-8 chaînons présentant 1-3 liaisons C-C non saturées.

4. Promoteur d'hydrate selon l'une quelconque des revendications 1 à 3, dans lequel la structure cyclique présentant N et S dans la Formule I est un cycle à 5-6 chaînons, et les atomes du squelette restants de la structure cyclique présentant N et S sont des atomes de C ;

   préférablement, le groupe -COO- est situé en position ortho par rapport à l'atome N du cycle dans la structure cyclique présentant N et S ;
   plus préférablement, le composant A est sélectionné parmi des composés de pénicilline présentant un groupe représenté par la Formule III ;

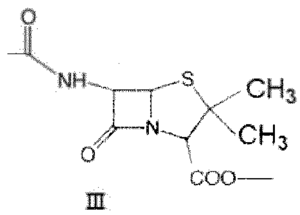

III

.

5. Promoteur d'hydrate selon l'une quelconque des revendications 1 à 4, dans lequel le composant A est sélectionné parmi un antibiotique de la famille des pénicillines et/ou un sel correspondant à l'antibiotique de la famille des pénicillines ; préférablement, le sel correspondant à l'antibiotique de la famille des pénicillines est au moins un sel sélectionné parmi le groupe constitué du sel de sodium, du sel de potassium et du sel d'ammonium ;

   plus préférablement, l'antibiotique de la famille des pénicillines est au moins un antibiotique sélectionné parmi le groupe constitué de la classe de la pénicilline G, de la classe de la pénicilline V, de la pénicilline résistante aux

enzymes, de la classe de l'ampicilline et de la pénicilline résistante aux Pseudomonas ;
plus préférablement encore, le composé de pénicilline est au moins un composé sélectionné parmi le groupe constitué de l'ampicilline sodique, de l'ampicilline, de la pénicilline potassique, de la carbénicilline sodique, de la pénicilline sodique et de l'oxacilline sodique.

**6.** Promoteur d'hydrate selon l'une quelconque des revendications 1 à 5, dans lequel le promoteur d'hydrate comprend en outre un adjuvant sélectionné parmi une gomme polysaccharidique et/ou un sel inorganique hydrosoluble ;

préférablement, un rapport massique du composant A à l'adjuvant est de 1 : (0,1-5); préférablement, le composant A est sélectionné parmi les composés de pénicilline, l'adjuvant est sélectionné parmi les sels inorganiques hydrosolubles ;
préférablement, un rapport massique du composé de pénicilline, du tensioactif et du sel inorganique hydrosoluble est de 1 : (0,1-5): (0,1-5), préférablement 1 : (0,1-1): (0,1-5);
plus préférablement, le sel inorganique hydrosoluble est au moins un sel sélectionné parmi le groupe constitué de $Na_2CO_3$, $K_2CO_3$, NaCl, KCl et $MgCl_2$.

**7.** Promoteur d'hydrate selon l'une quelconque des revendications 1 à 6, dans lequel le promoteur d'hydrate comprend en outre un antiagglomérant, dans lequel une structure moléculaire de l'antiagglomérant contenant un carbonyle et/ou un hydroxyle et une pluralité de structures cycliques ;

préférablement, l'antiagglomérant contient 3-4 cycles à plusieurs chaînons ;
plus préférablement, le cycle à plusieurs chaînons est un cycle à cinq chaînons ou un cycle à six chaînons ;
plus préférablement encore, le cycle à plusieurs chaînons est sélectionné parmi un phényle ou un groupe contenant au moins un élément parmi une liaison C-C non saturée et un groupe ester ;
plus préférablement encore, l'antiagglomérant est sélectionné parmi les composés organiques de statine, préférablement au moins un composé sélectionné parmi le groupe constitué de la pravastatine, de la lovastatine, de la simvastatine et de l'atorvastatine.

**8.** Promoteur d'hydrate selon la revendication 7, dans lequel l'antiagglomérant est contenu en une quantité de 1-10 parties en masse, préférablement 3-6 parties en masse, par rapport à une quantité totale de 1 partie en masse du composant A et du tensioactif et, facultativement, de l'adjuvant contenus dans le promoteur d'hydrate.

**9.** Promoteur d'hydrate selon l'une quelconque des revendications 1 à 8, dans lequel le promoteur d'hydrate comprend en outre un agent effervescent, qui est apte à libérer spontanément du gaz dans l'eau ;

préférablement, l'agent effervescent est utilisé en une quantité de 0,01-0,5 parties en masse, préférablement 0,02-0,3 parties en masse, par rapport à une quantité totale de 1 partie en masse du composant A et du tensioactif et, facultativement, de l'adjuvant contenus dans le promoteur d'hydrate ;
plus préférablement, l'agent effervescent comprend une source d'acide et une source alcaline ; préférablement, un rapport massique de la source d'acide et de la source alcaline est de 1 : (0,5-2);
plus préférablement encore, la source d'acide est au moins un composé sélectionné parmi le groupe constitué de l'acide citrique, de l'acide malique, de l'acide borique, de l'acide tartrique et de l'acide fumarique, et la source alcaline est sélectionnée parmi un carbonate basique et/ou un bicarbonate basique, préférablement au moins un composé sélectionné parmi le groupe constitué du bicarbonate de sodium, du carbonate de sodium, du carbonate de potassium et du bicarbonate de potassium.

**10.** Procédé de préparation d'un hydrate, comprenant : une mise en contact, dans un système aqueux, d'un gaz avec le promoteur d'hydrate selon l'une quelconque des revendications 1 à 9, dans des conditions de formation d'hydrate.

**11.** Procédé selon la revendication 10, dans lequel les conditions de formation d'hydrate comprennent : une température de -20 à 50°C ; une pression de 0,1-20 MPa.

**12.** Procédé selon la revendication 10 ou la revendication 11, dans lequel un rapport massique de la masse totale du composant A et du tensioactif et, facultativement, de l'adjuvant ajouté, par rapport à l'eau, est de (0,1-10):100.

**13.** Hydrate comprenant le promoteur d'hydrate selon l'une quelconque des revendications 1 à 9.

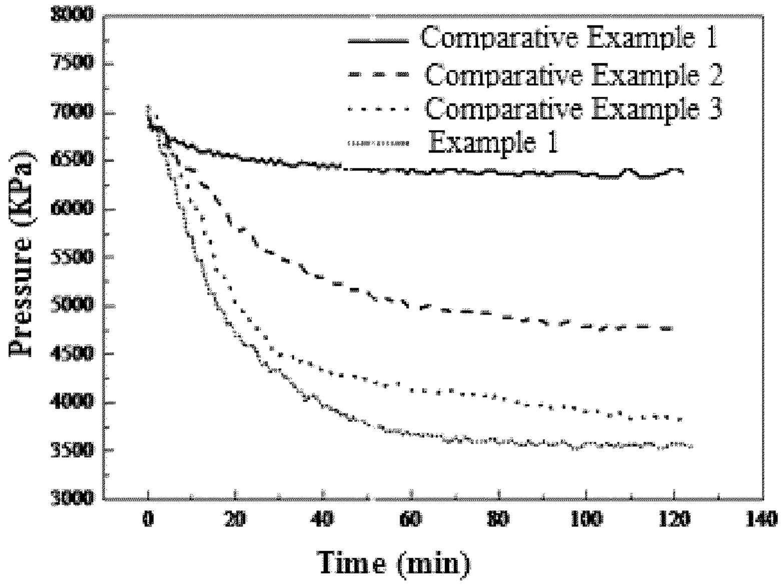
Comparative Example 1
Comparative Example 2
Comparative Example 3
Example 1
Pressure (KPa)
8000
7500
7000
6500
6000
5500
5000
4500
4000
3500
3000
0
20
40
60
80
100
120
140
Time (min)

FIG. 1

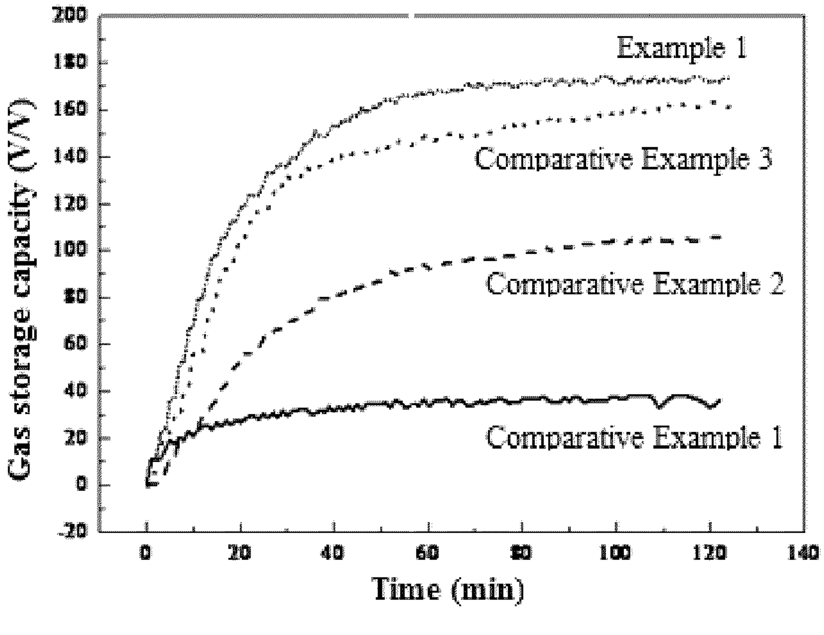
Example 1
Comparative Example 3
Comparative Example 2
Comparative Example 1
Gas storage capacity (V/V)
200
180
160
140
120
100
80
60
40
20
0
-20
0
20
40
60
80
100
120
140
Time (min)

FIG. 2

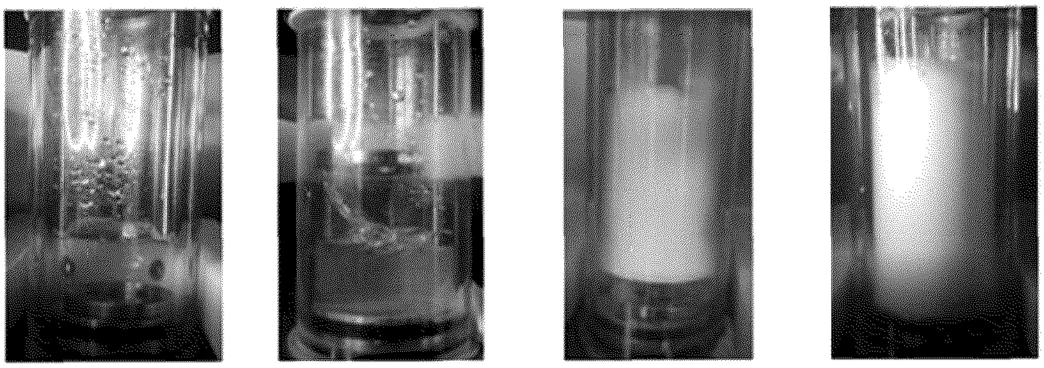

Example 1 Comparative Example 1 Comparative Example 2 Comparative Example 3

FIG. 3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 101672425 A
- CN 200810156914
- CN 103028353 A
- CN 201210544097
- CN 106634827 A
- CN 201610813750X
- CN ZL201110096579
- CN 02115154 A
- CN ZL201510501017 X